(19) Europäisches Patentamt
European Patent Office
Office européen des brevets

(11) Veröffentlichungsnummer: 0 326 916
A2

(12) # EUROPÄISCHE PATENTANMELDUNG

(21) Anmeldenummer: 89101242.9

(22) Anmeldetag: 25.01.89

(51) Int. Cl.4: C07D 401/04 , A61K 31/47 , C07D 471/04 , C07D 491/04 , C07D 405/14 , C07D 498/10 , C07D 519/00 , C07D 207/24 , C07D 211/42 , C07D 211/48 , C07D 491/10

(30) Priorität: 05.02.88 DE 3803478
29.04.88 DE 3814517

(43) Veröffentlichungstag der Anmeldung:
09.08.89 Patentblatt 89/32

(84) Benannte Vertragsstaaten:
AT BE CH DE ES FR GB GR IT LI NL SE

(71) Anmelder: BAYER AG

D-5090 Leverkusen 1 Bayerwerk(DE)

(72) Erfinder: Petersen, Uwe, Dr.
Auf dem Forst 4
D-5090 Leverkusen 1(DE)
Erfinder: Schenke, Thomas, Dr.
Muehlenstrasse 113
D-5060 Bergisch-Gladbach 2(DE)
Erfinder: Grohe, Klaus, Dr.
Am Wasserturm 10
D-5068 Odenthal(DE)
Erfinder: Schriewer, Michael, Dr.
Am Thelen Siefen 1 a
D-5068 Odenthal(DE)
Erfinder: Haller, Ingo, Dr.
Dornroeschenweg 4
D-5600 Wuppertal 1(DE)
Erfinder: Metzger, Karl Georg, Dr.
Pahlkestrasse 75
D-5600 Wuppertal 1(DE)
Erfinder: Endermann, Rainer, Dr.
In den Birken 152 a
D-5600 Wuppertal 1(DE)
Erfinder: Zeiler, Hans-Joachim, Dr.
Elsbeeker Strasse 46
D-5620 Velbert(DE)

(54) **Chinolon- und Naphthyridoncarbonsäurederivate, Verfahren zu ihrer Herstellung und sie enthaltende antibakterielle Mittel und Futterzusatzstoffe.**

(57) Die Erfindung betrifft Chinolon- und Naphthyridoncarbonsäure-Derivate der Formel (I)

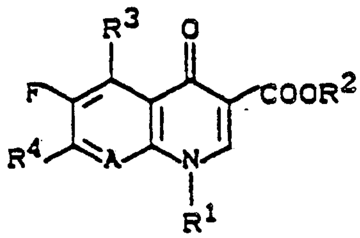

(I),

in welcher $R^1$, $R^2$, $R^3$, $R^4$ und A die in der Beschreibung angegebene Bedeutung haben, Verfahren zu ihrer Herstellung, sowie diese enthaltende antibakterielle Mittel und Futterzusatzstoffe.

EP 0 326 916 A2

# Chinolon- und Naphthyridoncarbonsäurederivate, Verfahren zu ihrer Herstellung und sie enthaltende antibakterielle Mittel und Futterzusatzstoffe

Die Erfindung betrifft Chinolon- und Naphthyridoncarbonsäure-Derivate, die in 7-Stellung durch einen cyclischen Aminrest substituiert sind, welcher ein quartäres Kohlenstoffatom trägt, Verfahren zu ihrer Herstellung, sowie diese enthaltende antibakterielle Mittel und Futterzusatzstoffe.

Es wurde gefunden, daß Chinolon- und Naphthyridoncarbonsäure-Derivate der Formel (I)

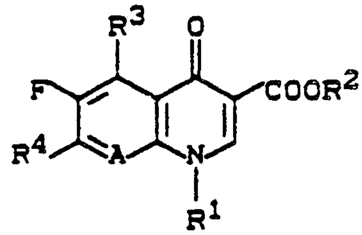 (I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl oder 2,4-Difluorphenyl,

$R^2$ für Wasserstoff, Alkyl mit 1 bis 4 Kohlenstoffatomen oder (5-Methyl-2-oxo-1,3-dioxol-4-yl)-methyl,

$R^3$ für Wasserstoff oder Amino,

$R^4$ für einen Rest der Formel

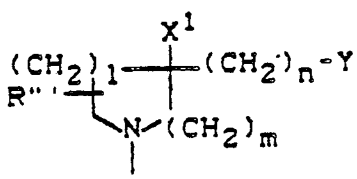 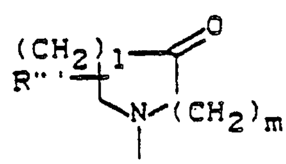,

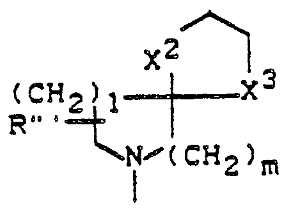,

worin

l für 0, 1 oder 2,

m für 1 oder 2 steht, wobei l + m gemeinsam 1, 2 oder 3 sein können,

n für 1 oder 2,

Y für 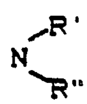,

OR, SR, Halogen oder Wasserstoff,

$X^1$ für 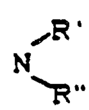,

OR, SR, Halogen, CN, $CONH_2$, COOH oder $C_1$-$C_4$-Alkyl steht,

$X^2$ und $X^3$ gleich oder verschieden sein können und für Sauerstoff, Schwefel, NH oder N-$CH_3$ stehen,

R für Wasserstoff, $C_1$-$C_3$-Alkyl oder $C_1$-$C_3$-Acyl,
R′ für Wasserstoff, $C_1$-$C_3$-Alkyl, Allyl oder Propargyl und
R″ für Wasserstoff, $C_1$-$C_3$-Alkyl oder $C_3$-$C_6$-Cycloalkyl steht,
wobei
R′ + R″ auch gemeinsam die Gruppen $-CH_2CH_2-O-CH_2CH_2-$ oder $-(CH_2)_k-$, in welcher k für 3, 4, oder 5 stehen kann, bedeutet,
R‴ für Wasserstoff oder $C_1$-$C_3$-Alkyl,
wobei 3-Amino-3-methyl-1-pyrrolidinyl für $R^4$ ausgenommen ist, und
A für N oder C-$R^5$ steht, worin
$R^5$ für Wasserstoff, Halogen wie Fluor oder Chlor, Methyl, Cyano oder Nitro steht oder auch gemeinsam mit $R^1$ eine Brücke der Struktur
$-O-CH_2-CH-CH_3$, $-S-CH_2-CH-CH_3$ oder $-CH_2-CH_2-CH-CH_3$
bilden kann,
und deren pharmazeutisch verwendbaren Hydrate und Säureadditionssalze sowie die Alkali-, Erdalkali-, Silber-und Guanidiniumsalze der zugrundeliegenden Carbonsäuren eine hohe antibakterielle Wirkung insbesondere im grampositiven Bereich aufweisen.

Sie eignen sich daher als Wirktoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen
$R^1$ für Ethyl, Isopropyl, Cyclopropyl, Vinyl, 2-Hydroxyethyl, 2-Fluorethyl, Amino, Methylamino, Phenyl, 4-Fluorphenyl oder 2,4-Difluorphenyl,
$R^2$ für Wasserstoff, Alkyl mit 1 bis 3 Kohlenstoffatomen oder (5-Methyl-2-oxo-1,3-dioxol-4-yl)-methyl,
$R^3$ für Wasserstoff oder Amino,
$R^4$ für einen Rest der Formel

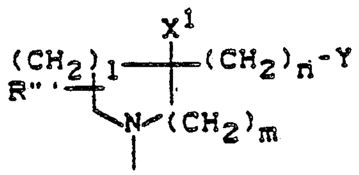

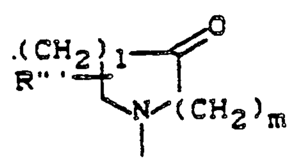

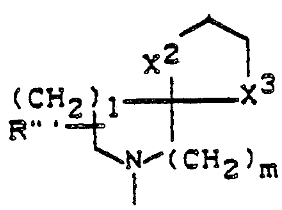
,

worin
l für 0, 1 oder 2,
m für 1 oder 2 steht, wobei l + m gemeinsam 1, 2 oder 3 sein können,
n für 1 oder 2,

Y für 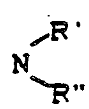 ,

OR, oder Wasserstoff,

$X^1$ für 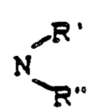 ,

OR, Fluor, Chlor oder $C_1$-$C_2$-Alkyl steht,
$X^2$ und $X^3$ gleich oder verschieden sein können und für Sauerstoff, Schwefel oder N-$CH_3$ stehen,

R für Wasserstoff, $C_1$-$C_2$-Alkyl oder Acetyl,
R′ für Wasserstoff oder $C_1$-$C_2$-Alkyl, und
R″ für Wasserstoff oder $C_1$-$C_2$-Alkyl steht, wobei
R′ + R″ auch gemeinsam die Gruppen $-CH_2CH_2-O-CH_2CH_2-$ oder $-(CH_2)_k$, in welcher k für 3, 4 oder 5 stehen kann, bedeutet,
R‴ für Wasserstoff oder $C_1$-$C_2$-Alkyl und
A für N oder C-$R^5$ steht, worin
$R^5$ für Wasserstoff, Halogen wie Fluor oder Chlor oder Methyl steht oder auch gemeinsam mit $R^1$ eine Brücke der Struktur
$-O-CH_2-\underset{|}{C}H-CH_3$ oder $-CH_2-CH_2-\underset{|}{C}H-CH_3$
bilden kann,
und deren pharmazeutisch verwendbaren Hydrate und Säureadditionssalze sowie die Alkali-, Erdalkali-, Silber-und Guanidiniumsalze.

Besonders bevorzugt sind diejenigen Verbindungen der Formel (I), in denen
$R^1$ für Ethyl, Isopropyl, Cyclopropyl, Vinyl, 2-Hydroxyethyl, 2-Fluorethyl, Amino, Methylamino, Phenyl, 4-Fluorphenyl oder 2,4-Difluorphenyl,
$R^2$ für Wassertoff oder Alkyl mit 1 oder 2 Kohlenstoffatomen,
$R^3$ für Wasserstoff,
$R^4$ für einen Rest der Formel

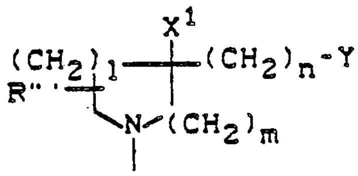

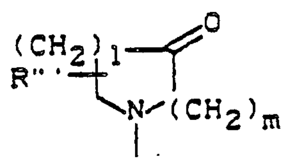

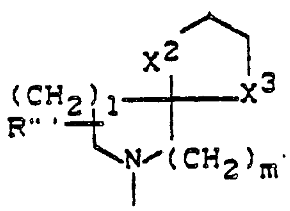 ,

worin
l für 0, 1 oder 2,
m für 1 oder 2 steht, wobei l + m gemeinsam 1, 2 oder 3 sein können,
n für 1,

Y für 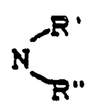 ,

OR oder Wasserstoff,

$X^1$ für 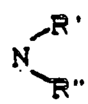 ,

OR, Chlor oder Methyl steht,
$X^2$ und $X^3$ gleich oder verschieden sein können und für Sauerstoff oder $N-CH_3$ stehen,
R für Wasserstoff oder Methyl,
R′ für Wasserstoff oder Methyl,
R″ für Wasserstoff oder Methyl und
R‴ für Wasserstoff oder Methyl steht und
A für N oder C-$R^5$ steht, worin

$R^5$ für Wasserstoff, Halogen, wie Fluor oder Chlor steht oder auch gemeinsam mit $R^1$ eine Brücke der Struktur

$-O-CH_2-CH-CH_3$
|

bilden kann,

und deren pharmazeutisch verwendbaren Hydrate und Säureadditionssalze sowie die Alkali-, Erdalkali-, Silber-und Guanidiniumsalze.

Weiterhin wurde gefunden, daß man die Verbindungen der Formel (I) erhält, wenn man Verbindungen der Formel (II)

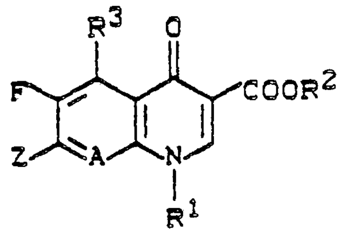

(II),

in welcher

Z für Fluor oder Chlor steht und

$R^1$, $R^2$, $R^3$ und A die oben angegebene Bedeutung haben, mit Verbindungen der Formel (III)

$R^4$-H (III),

in welcher

$R^4$ die oben angegebene Bedeutung hat, gegebenenfalls in Gegenwart von Säurefängern umsetzt.

Verbindungen der Struktur (Ia)

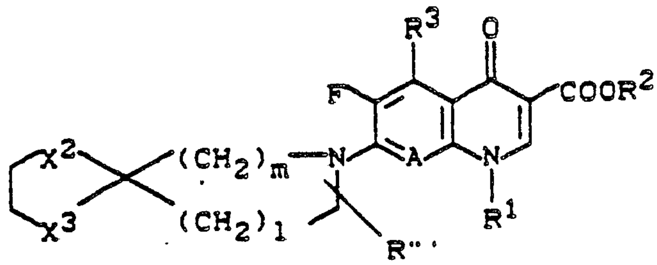

(Ia),

in welcher

$R^1$, $R^2$, $R^3$, R''', A, $X^2$, $X^3$, m und l die oben angegebene Bedeutung haben, können ebenfalls durch Umsetzung einer Verbindung der Formel (IV)

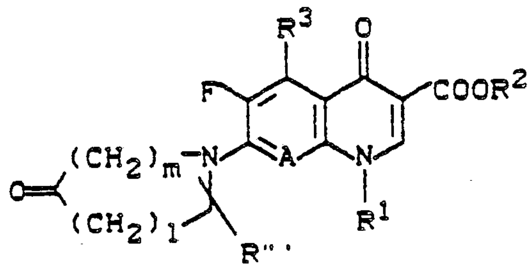

(IV),

in welcher

$R^1$, $R^2$, $R^3$, R''', A, m und l die oben angegebene Bedeutung haben, mit einer Verbindung der Formel (V)

$H-X^2-CH_2-CH_2-X^3-H$ (V),

in welcher

$X^2$ und $X^3$ die oben angegebene Bedeutung haben,

hergestellt werden.

Verwendet man beispielsweise 8-Chlor-1-cyclopropyl-6,7-difluor-1,4-dihydro-4-oxo-3-chinolincarbonsäure und 3-Ethylaminomethyl-3-hydroxy-pyrrolidin als Ausgangsstoffe, so kann der Reaktionsablauf durch das folgende Formelschema wiedergegeben werden:

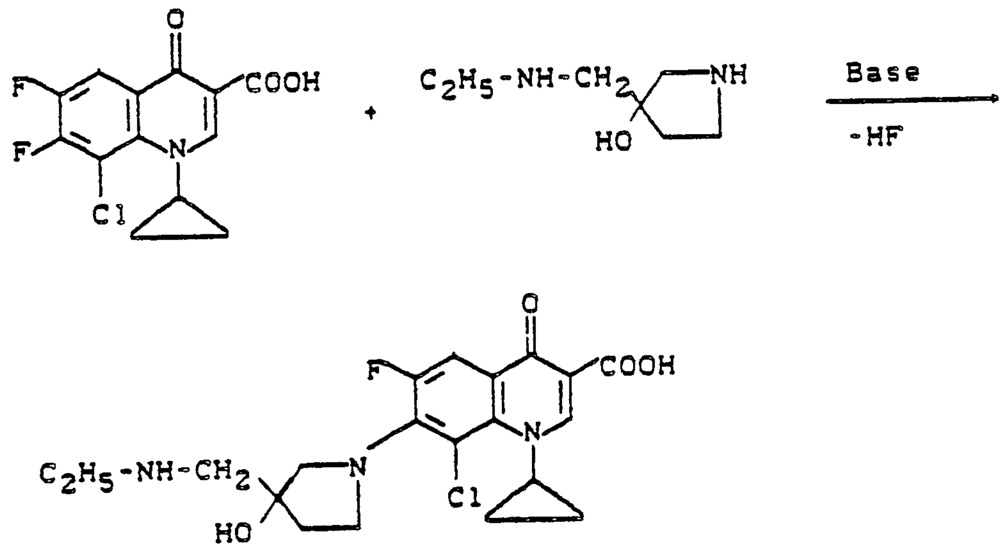


Die als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854),
1-Cyclopropyl-6,7-difluor-1,4-dihydro-4-oxo-3-chinolincarbonsäure (Europäische Patentanmeldung 113 091),
8-Chlor-1-cyclopropyl-6,7-difluor-1,4-dihydro-4-oxo-3-chinolincarbonsäure (Deutsche Patentanmeldung 3 420 743),
1-Cyclopropyl-6,7,8-trifluor-1,4-dihydro-4-oxo-3-chinolin-carbonsäure (Deutsche Patentanmeldung 3 318 145),
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
1-Cyclopropyl-7-chlor-6-fluor-1,4-dihydro-8-nitro-4-oxo-3-chinolincarbonsäure,
6,7-Difluor-1-(2-fluorethyl)-1,4-dihydro-4-oxo-3-chinolincarbonsäure,
6,7-Difluor-1,4-dihydro-1-methoxy-4-oxo-3-chinolincarbonsäure,
6,7-Difluor-1,4-dihydro-1-methylamino-4-oxo-3-chinolincarbonsäure,
6,7-Difluor-1,4-dihydro-4-oxo-1-phenyl-3-chinolincarbonsäure,
6,7-Difluor-1-(4-fluor-phenyl)-1,4-dihydro-4-oxo-3-chinolincarbonsäure,
6,7-Difluor-1-(3,4-difluor-phenyl)-1,4-dihydro-4-oxo-3-chinolincarbonsäure,
7-Chlor-1-cyclopropyl-6-fluor-1,4-dihydro-4-oxo-1,8-naphthyridin-3-carbonsäure,
1-Cyclopropyl-6,7,8-trifluor-1,4-dihydro-4-oxo-3-chinolincarbonsäureethylester (Deutsche Patentanmeldung 3 318 145),
9,10-Difluor-2,3-dihydro-3-methyl-7-oxo-7H-pyrido[1,2,3-de][1,4]benzoxacin-6-carbonsäure (Europäische Patentanmeldung 47 005),
8,9-Difluor-6,7-dihydro-5-methyl-1-oxo-1H,5H-benzo[i,j]chinolicin-2-carbonsäure,
7-Chlor-6-fluor-1-phenyl-1,4-dihydro-4-oxo-1,8-naphthyridin-3-carbonsäure (Europäische Patentanmeldung 153 580),
7-Chlor-6-fluor-1-(4-fluorphenyl)-1,4-dihydro-4-oxo-1,8-naphthyridin-3-carbonsäure (Europäische Patentanmeldung 153 580),
6,7,8-Trifluor-1,4-dihydro-1-methylamino-4-oxo-3-chinolincarbonsäure (Deutsche Patentanmeldung 3 409 922),
1-Amino-6,7,8-trifluor-1,4-dihydro-4-oxo-3-chinolincarbonsäure (Deutsche Patentanmeldung 3 409 922),
6,7,8-Trifluor-1,4-dihydro-1-dimethylamino-4-oxo-3-chinolincarbonsäure (Deutsche Patentanmeldung 3 409 922),

7-Chlor-6-fluor-1,4-dihydro-8-nitro-4-oxo-1-phenyl-3-chinolincarbonsäure,
7-Chlor-6-fluor-1-(4-fluorphenyl)-1,4-dihydro-8-nitro-4-oxo-3-chinolincarbonsäure,
6,7-Difluor-1-(4-fluorphenyl)-1,4-dihydro-8-methyl-4-oxo-3-chinolincarbonsäure,
6-Chlor-7-fluor-1-(4-fluorphenyl)-1,4-dihydro-4-oxo-3-chinolincarbonsäure (Europäische Patentanmeldung 131 839),
6-Chlor-7-fluor-1-(2,4-difluorphenyl)-1,4-dihydro-4-oxo-3-chinolincarbonsäure (Europäische Patentanmeldung 131 839),
6,7,8-Trifluor-1-(4-fluorphenyl)-1,4-dihydro-4-oxo-3-chinolincarbonsäure (Europäische Patentanmeldung 154 780),
6,7,8-Trifluor-1-(2,4-difluorphenyl)-1,4-dihydro-4-oxo-3-chinolincarbonsäure (Europäische Patentanmeldung 154 780),
6,7,8-Trifluor-1,4-dihydro-4-oxo-1-phenyl-3-chinolincarbonsäure (Europäische Patentanmeldung 154 780),
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure.

Die als Ausgangsverbindungen verwendeten Verbindungen der Formel (III) sind zum Teil neu und daher Gegenstand der Erfindung.

Ihre Herstellung kann nach verschiedenen Verfahren erfolgen:

1. Durch Umsetzung der am Stickstoff geschützten Spiro-oxirane (1) [J. Med. Chem. 30, 222 (1987); US-P 4 508 724; EP 189 370] mit Aminen (2) erfolgt Ringöffnung zu den Hydroxyaminen (3). Abspaltung der Schutzgruppe liefert Ausgangsverbindungen der Formel (IIIa):

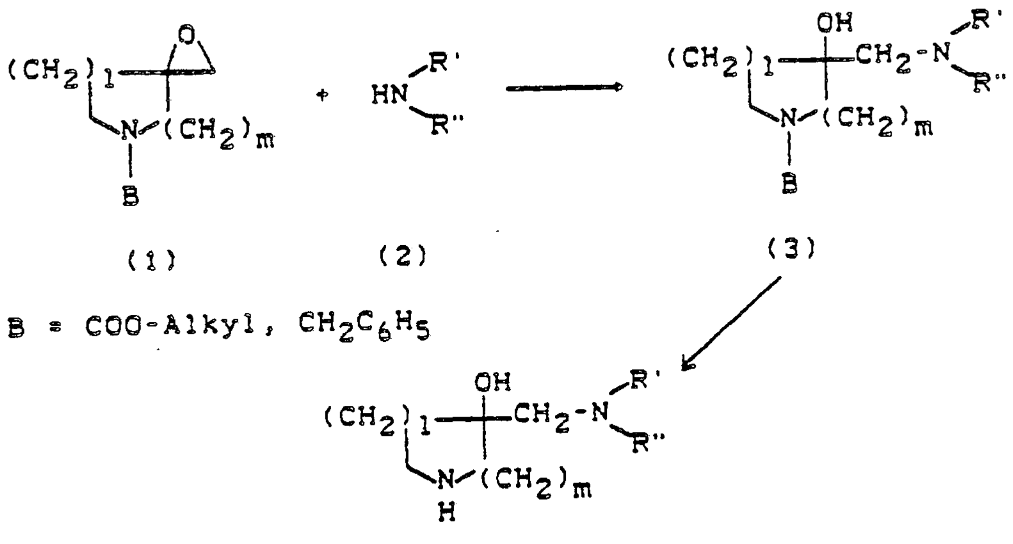

(1) (2) (3)

B = COO-Alkyl, $CH_2C_6H_5$

(IIIa)

2. Die Cyclisierung des Bernsteinsäureesters (4) [Tetrahedron Letters 46, 4561 (1973)] mit Benzylamin liefert den 1-Benzyl-3-hydroxy-5-oxo-pyrrolidin-3-carbonsäurealkylester (5), der nach Umsetzung mit einem Amin (2) zum Amid (6) reagiert. Nachfolgende Reduktion mit $LiAlH_4$ und hydrogenoly tische Abspaltung der Benzylgruppe liefert Ausgangsverbindungen der Formel (IIIb):

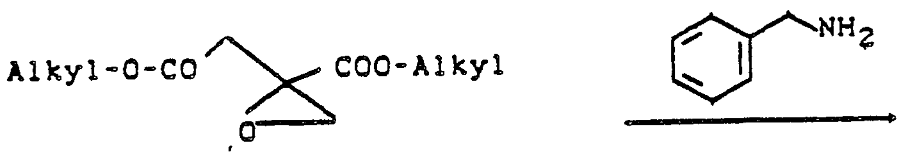

(4)

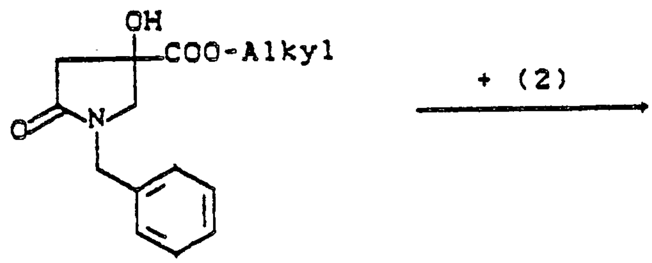


(5)

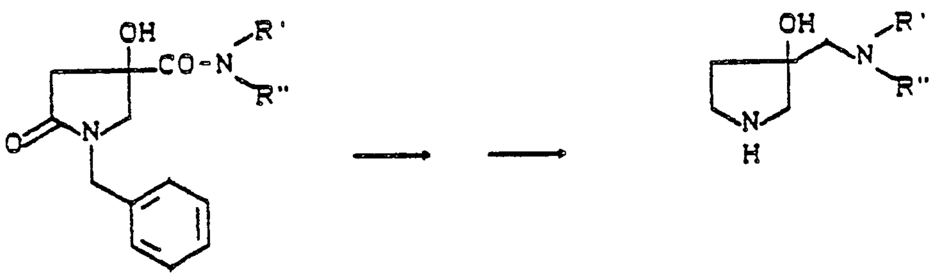

(6)  (IIIb)

3. Umsetzung der (1-Benzyl-3-hydroxy-2,5-dioxopyrrolidin-3-yl)-essigsäure (7) [Gazz. Chim. Ital. 24, 226 (1984)] zum Amid (8) und nachfolgende Reduktion mit $LiAlH_4$ und Abspaltung der Benzylgruppe liefert Ausgangsverbindungen der Formel (IIIc):

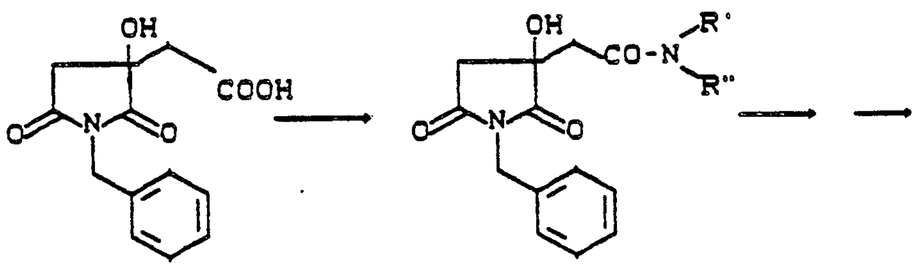

(7)  (8)

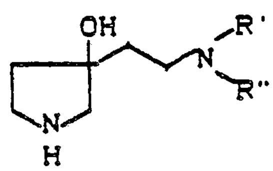

(IIIc)

4. 3-Hydroxy-3-methyl-pyrrolidin kann durch $LiAlH_4$-Reduktion von 4-Hydroxy-4-methyl-pyrrolidin-2-on [Zh. Org. Khim. 14, 7, S. 1420 (1978)] oder durch Debenzylierung von 1-Benzyl-3-hydroxy-3-methyl-pyrrolidin (EP 132 845) hergestellt werden.

5. Ausgehend von cyclischen Oxo-aminen (9), die am Stickstoff durch eine Schutzgruppe blockiert sind, können Ausgangsverbindungen der Formeln (IIId), (IIIe), (IIIf) aufgebaut werden [Acta Chem. Scand. B 34, 319 (1980)].

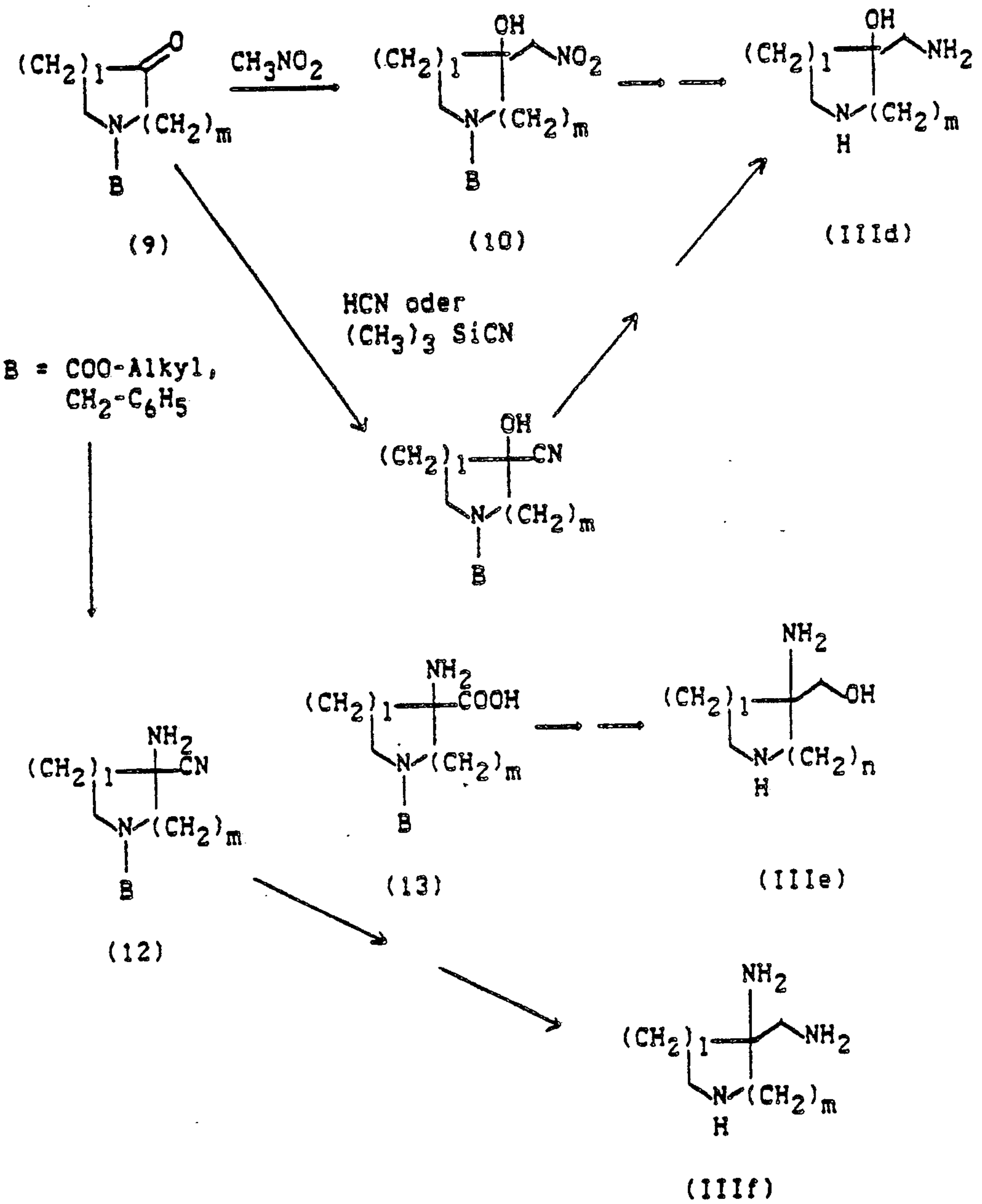


(9) (10) (IIId) (12) (13) (IIIe) (IIIf)

6. Die Hydroxygruppe der Hydroxyamine (IIIa) - (IIIf) kann alkyliert oder halogeniert werden.

7. Aus den cyclischen Oxoaminen (9) können Ketale, Thioketale oder Aminale hergestellt werden [Helv. Chim. Acta 50, 1289 (1967)].

Durch Umsetzung der am Stickstoff geschützten Spirooxirane (1) mit Trimethylsilylcyanid [J. Amer. Chem. Soc. 104, 5849 (1982)] können die Isonitrile (14) hergestellt werden, die durch Verseifen und Abspalten der Schutzgruppe zu den Ausgangsverbindungen der Formel (IIIg) umgesetzt werden können:

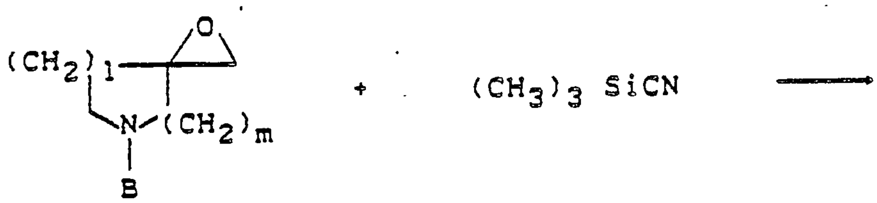


(1)

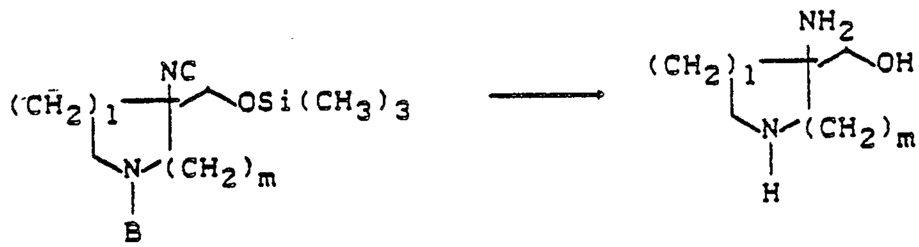

(14) (IIIg)

Als Beispiele für Ausgangsverbindungen der Formel (III) seien folgende Verbindungen genannt, wobei chirale Verbindungen sowohl als Racemate als auch als enantiomerenreine Stoffe eingesetzt werden können:

3-Aminomethyl-3-hydroxy-pyrrolidin,
3-Acetylaminomethyl-3-hydroxy-pyrrolidin,
3-tert.-Butoxycarbonylaminomethyl-3-hydroxy-pyrrolidin
3-Hydroxy-3-methylaminomethyl-pyrrolidin,
3-Ethylaminomethyl-3-hydroxy-pyrrolidin,
3-Hydroxy-3-propylaminomethyl-pyrrolidin,
3-Ethylaminomethyl-3-methoxy-pyrrolidin,
3-Ethoxy-3-ethylaminomethyl-pyrrolidin,
3-Chlor-3-ethylaminomethyl-pyrrolidin,
3-Ethylaminomethyl-3-fluor-pyrrolidin,
3-Ethylaminomethyl-3-methyl-pyrrolidin,
3-Ethylaminomethyl-3-mercapto-pyrrolidin,
3-Ethylaminomethyl-3-methylthio-pyrrolidin,
3-Acetoxy-3-ethylaminomethyl-pyrrolidin,
3-Dimethylaminomethyl-3-hydroxy-pyrrolidin,
3-Hydroxy-3-pyrrolidinomethyl-pyrrolidin,
3-Hydroxy-3-morpholinomethyl-pyrrolidin,
3-Amino-3-ethylaminomethyl-pyrrolidin,
3-Acetylamino-3-ethylaminomethyl-pyrrolidin,
3-Ethylaminomethyl-3-methylamino-pyrrolidin,
3-Dimethylamino-3-ethylaminomethyl-pyrrolidin,
3-Amino-3-hydroxymethyl-pyrrolidin,
3-Acetylamino-3-hydroxymethyl-pyrrolidin,
3-Amino-3-methoxymethyl-pyrrolidin,
3-tert.-Butoxycarbonylamino-3-methoxymethyl-pyrrolidin,
3-Amino-3-methylthiomethyl-pyrrolidin,
3-Amino-3-mercaptomethyl-pyrrolidin,
3-Cyclopropylaminomethyl-3-hydroxy-pyrrolidin,
3-Isopropylaminomethyl-3-hydroxy-pyrrolidin,
1,4-Dioxa-7-azaspiro[4.4]nonan,
1-Oxa-4,7-diazaspiro[4.4]nonan,
4-Methyl-1-oxa-4,7-diazaspiro[4.4]nonan,
1-Thia-4,7-diazaspiro[4.4]nonan,
1,4,7-Triazaspiro[4.4]nonan,
1,4-Dimethyl-1,4,7-triazaspiro[4.4]nonan.

Die Umsetzung von (II) mit (III) wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether, Acetonitril oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, Natriumhydrid, organische Amine und Amidine. Als besonders geeignet seien im einzelnen genannt: Triethylamin, 1,4-Diazabicyclo-[2,2,2]octan (DABCO), 1,8-Diazabicyclo[5,4,0]undec-7-en (DBU) oder überschüssiges Amin (III).

Die Reaktionstemperaturen können in einem größeren Bereich variiert werden. Im allgemeinen arbeitet man zwischen etwa 20 und 200° C, vorzugsweise zwischen 80 und 180° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode A setzt man auf 1 Mol der Carbonsäure (II) 1 bis 15 Mol, vorzugsweise 1 bis 6 Mol des Amins (III) ein.

Außer den in den Beispielen aufgeführten Verbindungen seien als neue Wirkstoffe im einzelnen genannt:

9-Fluor-2,3-dihydro-10-(3-hydroxy-3-methylaminomethyl-1-pyrrolidinyl)-3-methyl-7-oxo-7H-pyrido[1,2,3-de]-[1,4]benzoxacin-6-carbonsäure,

8-(3-Ethylaminomethyl-3-hydroxy-1-pyrrolidinyl)-9-fluor-6,7-dihydro-5-methyl-1-oxo-1H,5H-benzo[i,j]-chinolicin-2-carbonsäure,

und ferner die in folgender Tabelle aufgeführten Verbindungen.

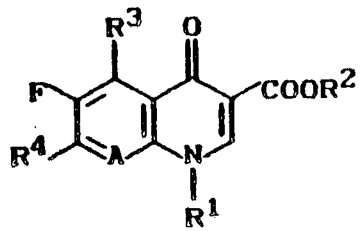

| Nr. | $R^1$ | $R^2$ | $R^3$ | $R^4$ | A |
|---|---|---|---|---|---|
| 1 | 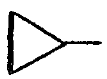 | H | H | 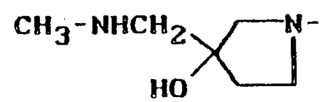 | CH |
| 2 | " | H | H | " | N |
| 3 | " | H | H | " | $C-CH_3$ |
| 4 | " | $C_2H_5$ | H | " | CCl |
| 5 | " | 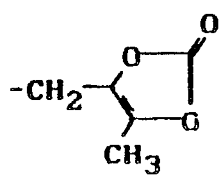 | H | " | CF |
| 6 | " | H | $NH_2$ | " | CF |
| 7 | $C_2H_5$ | H | H | " | CF |

| Nr. | $R^1$ | $R^2$ | $R^3$ | $R^4$ | A |
|---|---|---|---|---|---|
| 8 | 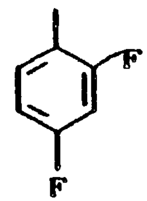 | H | H | 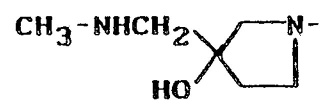 | CF |
| 9 | 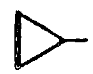 | H | H | 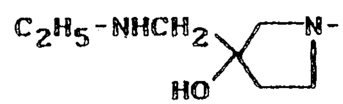 | N |
| 10 | " | H | H | " | $C-CH_3$ |
| 11 | " | H | $NH_2$ | " | CF |
| 12 | $C_2H_5$ | H | H | " | CF |
| 13 | 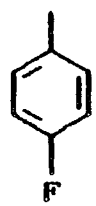 | H | H | " | N |
| 14 | 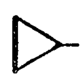 | $C_2H_5$ | H | " | CF |

| Nr. | $R^1$ | $R^2$ | $R^3$ | $R^4$ | A |
|---|---|---|---|---|---|
| 15 | 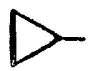 | 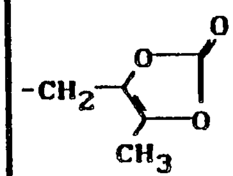 | H | 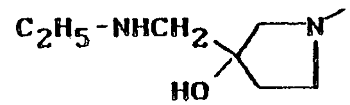 | CF |
| 16 | " | $C_2H_5$ | H | " | CCl |
| 17 | " | 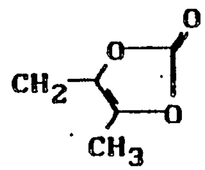 | H | " | CCl |
| 18 | " | H | H | 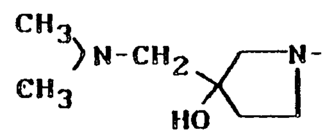 | CH |
| 19 | " | H | H | " | $C-CH_3$ |
| 20 | " | H | H | " | CCl |
| 21 | " | H | H | " | N |
| 22 | " | H | H | 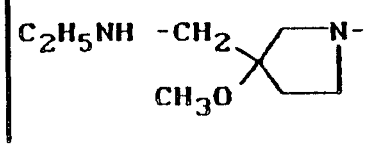 | CH |

| Nr. | $R^1$ | $R^2$ | $R^3$ | $R^4$ | A |
|---|---|---|---|---|---|
| 23 | 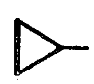 | H | H | 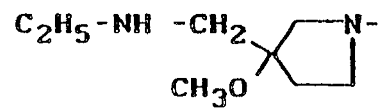 | CF |
| 24 | " | H | H | " | CCl |
| 25 | " | H | H | 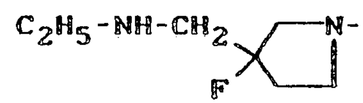 | CH |
| 26 | " | H | H | " | CF |
| 27 | " | H | H | " | CCl |
| 28 | " | H | H | " | N |
| 29 | " | H | H | 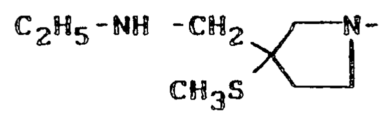 | CH |
| 30 | " | H | H | " | CF |

| Nr. | $R^1$ | $R^2$ | $R^3$ | $R^4$ | A |
|---|---|---|---|---|---|
| 31 | 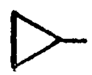 | H | H | 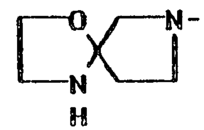 | CH |
| 32 | " | H | H | " | CF |
| 33 | " | H | H | " | CCl |
| 34 | " | H | H | " | N |
| 35 | " | H | H | " | $C-CH_3$ |
| 36 | " | H | H | 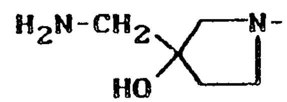 | CF |
| 37 | " | H | H | " | CCl |
| 38 | " | H | H | " | N |
| 39 | " | H | H | 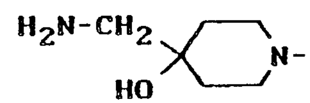 | CH |
| 40 | " | H | H | " | CF |
| 41 | " | H | H | " | CCl |

| Nr. | $R^1$ | $R^2$ | $R^3$ | $R^4$ | A |
|---|---|---|---|---|---|
| 42 | 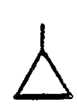 | H | H | 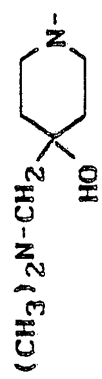 | CH |
| 43 | " | H | H | " | CF |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd; Bronchopneumonien, Fohlenlähme, puerperale und postpuerperale Infektionen, Salmonellose; Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, Aeromonas, Edwardsiella und Vibrio erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen,d eren Wirkstoffgehalt einem Bruchteil oder einem Vielfachen einer Einzeldosis entspricht.

Die Dosierungseinheiten können z.B. 1, 2, 3 oder 4 Einzeldosen oder 1/2, 1/3 oder 1/4 einer Einzeldosis

enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal intraperitoneal, lokal (Puder, Salbe, Tropfen) und zur Therapie von Infektionen in Hohlräumen, Körperhöhlen angewendet werden. Als geeignete Zubereitungen

kommen Injektionslösungen, Lösungen und Suspensionen für die orale Therapie, Gele, Aufgußformulierungen, Emulsionen, Salben oder Tropfen in Frage. Zur lokalen Therapie können ophthalmologische und dermatologische Formu lierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Keimwachstum zu erkennen war.

In der nachstehenden Tabelle sind die MHK-Werte einiger der erfindungsgemäßen Verbindungen im Vergleich zu Ciprofloxacin und Norfloxacin angegeben.

MHK-Werte (mg/1) bestimmt durch Agar-Verdünnungstest (Denley Multipoint Inoculator; Iso-Sensitest Agar, Oxoid)

| | Beispiel | 1 | 2 | 3 | 4 | 5 | 11 | 14 | 15 | Ciprofloxacin | Norfloxacin |
|---|---|---|---|---|---|---|---|---|---|---|---|
| Staphylococcus | FK 422 | 0,5 | 0,125 | 0,5 | 0,062 | 0,25 | 0,062 | 0,125 | 0,031 | 0,25 | 0,5 |
| aureus | 1756 | 0,5 | 0,125 | 0,5 | 0,062 | 0,125 | 0,062 | 0,125 | 0,031 | 0,25 | 0,5 |
| | 133 | 0,5 | 0,125 | 0,5 | 0,031 | 0,125 | 0,062 | 0,125 | 0,031 | 0,25 | 0,5 |
| Streptococcus faecalis (Enterococcus) | 27101 | 1 | 0,25 | 0,5 | 0,125 | 0,25 | 0,25 | 0,125 | 0,062 | 0,25 | 0,5 |
| | 9790 | 1 | 0,25 | 0,5 | 0,125 | 0,25 | 0,25 | 0,125 | 0,062 | 0,25 | 0,5 |

Herstellungsbeispiele für Zwischenverbindungen der Formel (III):

Beispiel A

3-Ethylaminomethyl-3-hydroxy-pyrrolidin

a) 5-Aza-1-oxaspiro[2,4]heptan-5-carbonsäureethylester

Man legt 23,5 g (107 mmol) Trimethylsulfoxoniumjodid und 3,3 g Natriumhydrid (80 % in Paraffinöl) vor und tropft bei 10 °C 80 ml absolutes Dimethylsulfoxid hinzu. Man rührt eine Stunde bei Raumtemperatur und tropft dann innerhalb von 15 Minuten 15,7 g (100 mmol) 3-Oxopyrrolidin-1-carbonsäureethylester [J. Med. Pharm. Chem. 5, 752 (1962)] in 20 ml absolutem Dimethylsulfoxid hinzu. Man rührt eine Stunde bei Raumtemperatur, gießt auf ein Gemisch von Eis und gesättigter Kochsalzlösung und extrahiert mit Diethylether. Die Etherlösungen werden mit Kochsalzlösung gewaschen, über $Na_2SO_4$ getrocknet, eingeengt und destilliert.
Ausbeute: 6 g
Siedepunkt: 80 °C/0,15 mbar

b) 3-Ethylaminomethyl-3-hydroxypyrrolidin-1-carbonsäureethylester

Man tropft 8 g (46,7 mmol) 5-Aza-1-oxaspiro[2,4]heptan-5-carbonsäureethylester zu 50 ml Ethylamin-Lösung (50 % in Wasser) und rührt über Nacht bei Raumtemperatur. Man engt ein und destilliert den Rückstand.
Ausbeute: 8 g
Siedepunkt: 130 °C/0,05 mbar

c) 3-Ethylaminomethyl-3-hydroxypyrrolidin

Man erhitzt 7,7 g (35,6 mmol) 3-Ethylaminomethyl-3-hydroxypyrrolidin-1-carbonsäureethylester mit 22 g $Ba(OH)_2 \bullet 8H_2O$ in 220 ml Wasser über Nacht unter Rückfluß. Man saugt von $BaCO_3$ ab und engt ein. Der Rückstand wird fünfmal mit je 100 ml Dioxan ausgekocht, die Dioxanlösung eingeengt und destilliert.
Ausbeute: 4,2 g
Siedepunkt: 70-75 °C/0,1 mbar

Beispiel B

3-Aminomethyl-3-hydroxy-pyrrolidin

a) 3-Aminomethyl-1-benzyl-3-hydroxypyrrolidin

Man tropft 9,7 g (51,3 mmol) 5-Benzyl-5-aza-1-oxaspiro[2,4]heptan (US-Patent 4 508 724) zu 50 ml Ammoniak-Lösung (25 %) und rührt über Nacht bei Raumtemperatur. Dann engt man den Ansatz ein und destilliert den Rückstand.
Ausbeute: 4,4 g
Siedepunkt: 134 °C/0,4 mbar

b) 3-Aminomethyl-3-hydroxypyrrolidin

Man hydriert 3,9 g (18,9 mmol) 3-Aminomethyl-1-benzyl-3-hydroxypyrrolidin in 25 ml Methanol mit 1 g

Palladium-Aktivkohle (10 %) bei 90 °C und 95 bar. Der Katalysator wird abfiltriert, das Filtrat eingeengt und der Rückstand destilliert.
Ausbeute: 1,2 g
Siedepunkt: 80 °C/0,14 mbar

Beispiel C

3-Ethylaminomethyl-3-hydroxy-pyrrolidin

a) 1-Benzyl-3-ethylaminomethyl-3-hydroxy-pyrrolidin

Man tropft 10,2 g (53,9 mmol) 5-Benzyl-5-aza-1-oxaspiro[2,4]heptan zu 60 ml wäßriger Ethylaminlösung (50 %) und rührt über Nacht bei Raumtemperatur. Dann engt man den Ansatz ein und destilliert den Rückstand.
Ausbeute: 10,7 g
Siedepunkt: 120 °C/0,18 mbar

b) 3-Ethylaminomethyl-3-hydroxy-pyrrolidin

Man hydriert 10 g (42,7 mmol) 1-Benzyl-3-ethylaminomethyl-3-hydroxypyrrolidin in 60 ml Methanol mit 2 g Palladium-Aktivkohle (10 %) bei 92 °C und 107 bar. Der Katalysator wird abfiltriert, das Filtrat eingeengt und der Rückstand destilliert.
Ausbeute: 4,8 g
Siedepunkt: 74 °C/0,08 mbar

Beispiel D

3-Hydroxy-3-methylaminomethyl-pyrrolidin-Dihydrochlorid

a) 1-Benzyl-3-hydroxy-3-methylaminomethyl-pyrrolidin-Dihydrochlorid.

Man tropft 10,2 g (58,3 mmol) 5-Benzyl-5-aza-1-oxaspiro[2,4]heptan zu 70 ml wäßriger Methylaminlösung (30 %) und rührt über Nacht bei Raumtemperatur. Dann engt man den Ansatz ein und destilliert den Rückstand.
Ausbeute: 8,8 g
Siedepunkt: 145 °C/0,35 mbar

Das Destillat wird in verdünnter Salzsäure gelöst und die Lösung eingeengt. Der kristalline Rückstand wird mit Isopropanol verrieben, abgesaugt und getrocknet.
Ausbeute: 7,3 g
Schmelzpunkt: 202 °C

b) 3-Hydroxy-3-methylaminomethyl-pyrrolidin-Dihydrochlorid

Man hydriert 6,9 g (23,5 mmol) 1-Benzyl-3-hydroxy3-methylaminomethyl-pyrrolidin-Dihydrochlorid in 100 ml Methanol an 2 g Palladium-Aktivkohle (10 %) bei 80 °C und 100 bar. Man saugt den Katalysator ab, engt die Lösung ein und verreibt den Rückstand mit Butanol. Das kristalline Salz wird angesaugt, mit Aceton gewaschen und getrocknet.
Ausbeute: 4 g
Schmelzpunkt: 231-232 °C

Beispiel E

3-Cyclopropylaminomethyl-3-hydroxy-pyrrolidin-Dihydrochlorid

a) 1-Benzyl-3-cyclopropylaminomethyl-3-hydroxy-pyrrolidin-Dihydrochlorid

Man tropft 9,7 g (51,3 mmol) 5-Benzyl-5-aza-1-oxaspiro[2,4]heptan zu 9,7 g (0,17 mol) Cyclopropylamin in 40 ml Wasser und rührt über Nacht bei Raumtemperatur. Dann engt man den Ansatz ein und destilliert den Rückstand.
Ausbeute: 8 g
Siedepunkt: 130° C/0,08 mbar
Das Destillat wird in verdünnter Salzsäure gelöst und die Lösung eingeengt. Der kristallisierende Rückstand wird mit Aceton verrieben, abgesaugt und getrocknet.
Ausbeute: 8,3 g
Schmelzpunkt: 182-184° C

b) 3-Cyclopropylaminomethyl-3-hydroxy-pyrrolidin-Dihydrochlorid

Man hydriert 7,9 g (24,7 mmol) 1-Benzyl-3-cyclopropylaminomethyl-3-hydroxypyrrolidin-Dihydrochlorid in 100 ml Methanol an 2 g Palladium-Aktivkohle (10 %) bei 50° C und 100 bar. Man saugt ab, engt ein und verreibt den Rückstand mit Butanol. Das kristalline Salz wird abgesaugt, mit Aceton gewaschen und getrocknet.
Ausbeute: 3,4 g

Beispiel F

3-Aminomethyl-3-hydroxy-piperidin

a) 5-Aza-1-oxaspiro[2,5]octan-5-carbonsäuremethylester

Man legt 23,5 g (107 mmol) Trimethylsulfoxoniumjodid und 3,4 g (100 mmol) NaH (80 % in Paraffinöl) vor und tropft bei 10° C 80 ml absolutes Dimethylsulfoxid hinzu. Man rührt eine Stunde bei Raumtemperatur und tropft dann innerhalb von 15 Minuten 15,8 g (100 mmol) 3-Piperidon-1-carbonsäuremethylester [Acta Chem. Scand. B 30, (1976), Seite 884] hinzu. Man rührt eine Stunde bei Raumtemperatur, gießt auf ein Gemisch von Eis und gesättigter Kochsalzlösung und extrahiert mit Diethylether. Die Etherlösung wird mit Kochsalzlösung gewaschen, über $Na_2SO_4$ getrocknet, eingeengt und destilliert.
Ausbeute: 8 g
Siedepunkt: 68° C/0,15 mbar

b) 3-Aminomethyl-3-hydroxy-piperidin-1-carbonsäuremethylester

Man tropft 9,1 g (53,2 mmol) 5-Aza-1-oxaspiro[2,5]octan-5-carbonsäuremethylester zu 50 ml Ammoniak-Lösung (25 %) und rührt über Nacht bei Raumtemperatur. Dann engt man ein und destilliert den Rückstand.
Ausbeute: 5,6 g
Siedepunkt: 103° C/0,1 mbar

c) 3-Aminomethyl-3-hydroxy-piperidin

Man erhitzt 5,1 g (27,1 mmol) 3-Aminomethyl-3-hydroxy-piperidin-1-carbonsäuremethylester mit 15,8 g

$Ba(OH)_2 \bullet 8H_2O$ in 150 ml Wasser über Nacht unter Rückfluß. Man saugt von $BaCO_3$ ab und engt ein. Der Rückstand wird fünfmal mit je 70 ml Dioxan ausgekocht, die Dioxanlösungen eingeengt und destilliert.
Ausbeute: 1,8 g
Siedepunkt: 63° C/0,05 mbar

Beispiel G

3-Hydroxy-3-methylaminomethyl-piperidin

a) 3-Hydroxy-3-methylaminomethyl-piperidin-1-carbonsäuremethylester

Man tropft 9,3 g (54,3 mmol) 5-Aza-1-oxaspiro[2,5]octan-5-carbonsäuremethylester zu 50 ml Methylamin-Lösung (25 % in Wasser) und rührt über Nacht bei Raumtemperatur. Dann engt man ein und destilliert den Rückstand.
Ausbeute: 9,2 g
Siedepunkt: 83-95° C/0,1 mbar

b) 3-Hydroxy-3-methylaminomethyl-piperidin

Man erhitzt 8,7 g (43 mmol) 3-Hydroxy-3-methylaminomethyl-piperidin-1-carbonsäuremethylester mit 24 g $Ba(OH)_2 \bullet 8H_2O$ in 240 ml Wasser über Nacht unter Rückfluß. Man saugt von $BaCO_3$ ab und engt ein. Der Rückstand wird fünfmal mit je 100 ml Dioxan ausgekocht, die Dioxanlösung eingeengt und destilliert.
Ausbeute: 4,2 g
Siedepunkt: 56° C/0,05 mbar

Beispiel H

3-Ethylaminomethyl-3-hydroxy-piperidin

a) 3-Ethylaminomethyl-3-hydroxy-piperidin-1-carbonsäuremethylester

Man tropft 9,3 g (54,3 mmol) 5-Aza-1-oxaspiro[2,5]octan-5-carbonsäuremethylester zu 50 ml Ethylamin-Lösung (50 %ig in Wasser) und rührt über Nacht bei Raumtemperatur. Dann engt man ein und destilliert den Rückstand.
Ausbeute: 11,2 g
Siedepunkt: 104-108° C/0,2 mbar

b) 3-Ethylaminomethyl-3-hydroxy-piperidin

Man erhitzt 10 g (46,2 mmol) 3-Ethylaminomethyl-3-hydroxy-piperidin-1-carbonsäuremethylester mit 28,5 g $Ba(OH)_2 \bullet 8H_2O$ in 280 ml Wasser über Nacht unter Rückfluß. Man saugt von $BaCO_3$ ab und engt ein. Der Rückstand wird fünfmal mit je 120 ml Dioxan ausgekocht, die Dioxanlösung eingeengt und destilliert.
Ausbeute: 4,5 g
Siedepunkt: 70° C/0,09 mbar

Beispiel I

3-Aminomethyl-4-hydroxy-piperidin-Dihydrochlorid

a) 4-Aminomethyl-4-hydroxy-piperidin-1-carbonsäureethylester

Man tropft 13,4 g (72,3 mmol) 6-Aza-1-oxaspiro[2,5]octan-6-carbonsäureeethylester (Europäische Patentanmeldung 189 370) zu 60 ml Ammoniak-Lösung (25 %) und rührt über Nacht bei Raumtemperatur. Dann engt man ein und destilliert.
Ausbeute: 8,1 g
Siedepunkt: 110-130° C/0,4 mbar

b) 4-Aminomethyl-4-hydroxy-piperidin-Dihydrochlorid

Man erhitzt 1 g (4,9 mmol) 4-Aminomethyl-4-hydroxy-piperidin-1-carbonsäureeethylester mit 10 ml konzentrierter Salzsäure über Nacht unter Rückfluß. Man engt ein, verreibt die Kristalle mit Aceton, saugt ab und trocknet in Vakuumexsikkator über $P_4O_{10}$.
Ausbeute: 1 g
Schmelzpunkt: 230-233° C

Beispiel J

4-Hydroxy-4-methylaminomethyl-piperidin-Dihydrochlorid

a) 4-Hydroxy-4-methylaminomethyl-piperidin-1-carbonsäureethylester

Man tropft 5,2 g (28 mmol) 6-Aza-1-oxaspiro[2,5]octan-6-carbonsäureeethylester zu 30 ml Methylamin-Lösung (25 % in Wasser) und rührt über Nacht bei Raumtemperatur. Dann engt man ein und kristallisiert aus Waschbenzin um (hygroskopische Kristalle).
Ausbeute: 3,3 g

b) 4-Hydroxy-4-methylaminomethyl-piperidin-Dihydrochlorid

Man erhitzt 3 g (13,9 mmol) 4-Hydroxy-4-methylaminomethyl-piperidin-1-carbonsäureeethylester mit 30 ml konzentrierter Salzsäure über Nacht unter Rückfluß. Man engt ein, verrührt die Kristalle mit Aceton, saugt ab und trocknet in Vakuumexsikkator über $P_4O_{10}$.
Ausbeute: 2,7 g
Schmelzpunkt: 236-238° C

Beispiel K

4-Dimethylaminomethyl-4-hydroxy-piperidin-Dihydrochlorid

a) 4-Dimethylaminomethyl-4-hydroxy-piperidin-1-carbonsäureethylester

Man tropft 5,2 g (28 mmol) 6-Aza-1-oxaspiro[2,5]octan-6-carbonsäureeethylester zu 30 ml Dimethylamin-Lösung (40 % in Wasser) und rührt über Nacht bei Raumtemperatur. Dann engt man ein und destilliert.
Ausbeute: 5,2 g
Siedepunkt: 150-155° C/3 mbar

b) 4-Dimethylaminomethyl-4-hydroxy-piperidin-Dihydrochlorid

Man erhitzt 4,5 g (19,4 mmol) 4-Dimethylaminomethyl-4-hydroxy-piperidin-1-carbonsäureethylester mit 35 ml konzentrierter Salzsäure über Nacht unter Rückfluß. Man engt ein, verreibt die Kristalle mit Aceton, saugt ab und trocknet in Vakuumexsikkator über $P_4O_{10}$.
Ausbeute: 4,1 g
Schmelzpunkt: 224-227° C .

Beispiel L

4-Ethylaminomethyl-4-hydroxy-piperidin-Dihydrochlorid

a) 4-Ethylaminomethyl-4-hydroxy-piperidin-1-carbonsäureethylester

Man tropft 5,7 g (30,8 mmol) 6-Aza-1-oxaspiro[2,5]octan-6-carbonsäureethylester zu 30 ml Ethylamin-Lösung (50 % in Wasser) und rührt über Nacht bei Raumtemperatur. Dann engt man ein und destilliert.
Ausbeute: 5,5 g
Siedepunkt: 100-104° C/0.01 mbar

b) 4-Ethylaminomethyl-4-hydroxy-piperidin-Dihydrochlorid

Man erhitzt 4,6 g (20 mmol) 4-Ethylaminomethyl-4-hydroxypiperidin-1-carbonsäureethylester mit 35 ml konzentrierter Salzsäure über Nacht unter Rückfluß. Man engt ein, verreibt die Kristalle mit Aceton, saugt ab und trocknet in Vakuumexsikkator über $P_4O_{10}$.
Ausbeute: 4,2 g
Schmelzpunkt: 225-229° C

Beispiel M

3-Hydroxy-3-methylpyrrolidin

Man löst 15 g (78,4 mmol) 1-Benzyl-3-hydroxy-3-methylpyrrolidin (Europäische Patentanmeldung 132 845) in 150 ml Ethanol und hydriert an 2 g Palladium-Aktivkohle (10 % Pd) bei 90° C und 100 bar. Anschließend saugt man den Katalysator ab, engt das Filtrat ein und destilliert den Rückstand.
Ausbeute: 4,6 g
Siedepunkt: 60-64° C/0,08 mbar

Beispiel N

3-Dimethylaminomethyl-3-hydroxy-pyrrolidin

a) 1-Benzyl-3-dimethylaminomethyl-3-hydroxy-pyrrolidin

Man tropft 9,6 g (50 mmol) 5-Benzyl-5-aza-1-oxaspiro[2,4]heptan zu 50 ml Dimethylaminlösung (50 %) und rührt über Nacht bei Raumtemperatur. Dann engt man den Ansatz ein und destilliert den Rückstand.
Ausbeute: 10,3 g
Siedepunkt: 94° C / 0,05 mbar

b) 3-Dimethylaminomethyl-3-hydroxy-pyrrolidin

Man hydriert 9,4 g (39 mmol) 1-Benzyl-3-dimethylaminomethyl-3-hydroxy-pyrrolidin in 60 ml Methanol mit 2 g Palladium-Aktivkohle (5 %) bei 100 °C und 90 bar. Der Katalysator wird abfiltriert, das Filtrat eingeengt und der Rückstand destilliert.
Ausbeute: 4,3 g
Siedepunkt 51 °C / 0,06 mbar.

Beispiel O

3-Hydroxy-3-methoxymethyl-pyrrolidin

a) 1-Benzyl-3-hydroxy-3-methoxymethyl-pyrrolidin

Man erhitzt 26,8 g (0,14 mol) 5-Benzyl-1-oxa-5-azaspiro[2,4]heptan mit 2,6 ml (14 mmol) 30 %iger Natriummethylatlösung in 200 ml absolutem Methanol über Nacht unter Rückfluß. Man engt ein und destilliert.
Ausbeute: 25,6 g (83 % der Theorie)
Siedepunkt: 107-112 °C/0,15 mbar

b) 3-Hydroxy-3-methoxymethyl-pyrrolidin

Man hydriert 10 g (45 mmol) 1-Benzyl-3-hydroxy-3-methoxymethyl-pyrrolidin mit 3 g Pd-Aktivkohle (10 % Pd) in 200 ml Methanol bei 100 °C und 100 bar. Der Katalysator wird abgesaugt, das Filtrat eingeengt und der Rückstand destilliert.
Ausbeute: 4,7 g (80 % der Theorie)
Siedepunkt: 65 °C/0,4 mbar

Beispiel P

3-tert.-Butoxycarbonylaminomethyl-3-hydroxy-pyrrolidin

a) 1-Benzyl-3-tert.-butoxycarbonylaminomethyl-3-hydroxy-pyrrolidin

Man löst 3,2 g (80 mmol) NaOH in 40 ml Wasser, setzt 15,6 g (75 mmol) 3-Aminomethyl-1-benzyl-3-hydroxy-pyrrolidin und 50 ml tert.-Butanol hinzu und tropft bei Raumtemperatur 17,7 g (79 mmol) Dikohlensäure-di-tert.-butylester hinzu. Nach Rühren über Nacht bei Raumtemperatur saugt man ab, wäscht die Kristalle mit $CH_2Cl_2$ und extrahiert das Filtrat mit $CH_2Cl_2$. Die Extrakte werden über $K_2CO_3$ getrocknet, eingeengt, und der Rückstand aus Diisopropylether umkristallisiert.
Ausbeute: 19,1 g (83 % der Theorie)
Schmelzpunkt: 117-119 °C

b) 3-tert.-Butoxycarbonylaminomethyl-3-hydroxy-pyrrolidin

Man löst 18,7 g (61 mmol) 1-Benzyl-3-tert.-butoxycarbonylaminomethyl-3-hydroxy-pyrrolidin in 120 ml Methanol und hydriert an 3 g 5 %iger Pd-Aktivkohle bei 90 °C und 100 bar. Man filtriert den Katalysator ab, engt das Filtrat ein und kristallisiert den Rückstand aus Essigsäureethylester um.
Ausbeute: 9,2 g (70 % der Theorie)
Schmelzpunkt: 124-127 °C

Beispiel Q

3-Methoxy-3-methylaminomethyl-pyrrolidin

a) 1-Benzyl-3-benzylmethylaminomethyl-3-hydroxy-pyrrolidin

Zu 15,6 ml (0,115 mol) Benzylmethylamin in 300 ml Wasser tropft man 18,2 g (95 mmol) 5-Benzyl-1-oxa-5-azaspiro[2,4]heptan und rührt 15 Stunden bei Raumtemperatur. Man extrahiert mit $CH_2Cl_2$, trocknet die Extrakte mit $K_2CO_3$, engt ein und destilliert bis 160° C (Ölbadtemperatur) an.
Rohausbeute: 27,1 g
GC-Gehalt: 100 %

b) 1-Benzyl-3-benzylmethylaminomethyl-3-methoxy-pyrrolidin

26 g (83 mmol) rohes 1-Benzyl-3-benzylmethylaminomethyl-3-hydroxypyrrolidin in 50 ml absolutem Tetrahydrofuran tropft man zu 4 g 80 %igen Natriumhydrid in 100 ml absolutem Tetrahydrofuran und erhitzt dabei unter Rückfluß. Nach dem Ende der Wasserstoffentwicklung tropft man langsam 12,4 g (87 mmol) Methyliodid hinzu und erhitzt anschließend über Nacht unter Rückfluß. Man gießt auf Eiswasser, extrahiert mit Toluol, trocknet über $K_2CO_3$, engt ein und destilliert.
Ausbeute: 16,5 g
Siedebereich: 140-173° C/0,1-0,23 mbar
Nach erneuter Destillation:
Ausbeute: 9,1 g (26 % der Theorie)
GC-Gehalt: 80 %
Siedepunkt: 141° C/0,07 mbar

c) 3-Methoxy-3-methylaminomethyl-pyrrolidin

Man löst 8,4 g (20 mmol) 80 %iges 1-Benzyl-3-benzylmethylaminomethyl-3-methoxy-pyrrolidin in 100 ml Methanol, setzt 4,4 ml konzentrierte Salzsäure hinzu und hydriert an 4 g 10 %iger Pd-Aktivkohle bei 80° C und 120 bar. Man filtriert den Katalysator ab, engt ein, setzt eine Lösung von 3 g KOH in 50 ml Methanol hinzu, filtriert KCl ab und engt ein. Der Rückstand wird erneut in $CHCl_3$ aufgenommen, filtriert, eingeengt und destilliert.
Ausbeute: 1,7 g (59 % der Theorie)
Siedepunkt: 33° C/0,08 mbar

Beispiel 1

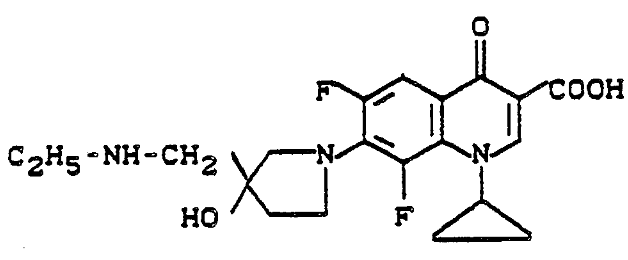

Eine Mischung aus 1,4 g (5 mmol) 1-Cyclopropyl-6,7,8-trifluor-1,4-dihydro-4-oxo-3-chinolincarbonsäure, 1,1 g (10 mmol) 1,4-Diazabicyclo[2.2.2]octan und 0,8 g (5,5 mmol) 3-Ethylaminomethyl-3-hydroxy-pyrrolidin in 10 ml Acetonitril und 5 ml Dimethylformamid wird 1 Stunde unter Rückfluß erhitzt. Die Suspension wird im Vakuum eingeengt, der Rückstand mit Wasser gut verrührt, der zurückbleibende Niederschlag abgesaugt, mit Wasser gewaschen, getrocknet und aus Glykolmonomethylether umkristallisiert.

Ausbeute: 1,2 g (59 % der Theorie) 1-Cyclopropyl-7-(3-ethylaminomethyl-3-hydroxy-1-pyrrolidinyl)-6,8-difluor-1,4-dihydro-4-oxo-3-chinolincarbonsäure vom Schmelzpunkt 230-232° (unter Zersetzung).

Beispiel 2

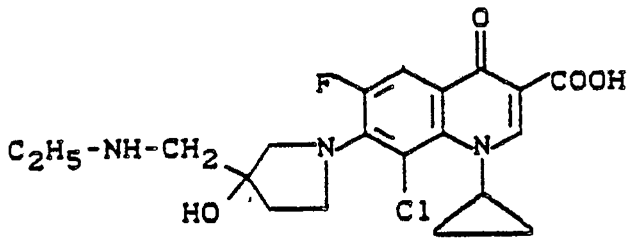

x HCl

1,5 g (5 mmol) 8-Chlor-1-cyclopropyl-6,7-difluor-1,4-dihydro-4-oxo-3-chinolincarbonsäure werden in 10 ml Acetonitril und 5 ml Dimethylformamid mit 1,1 g (10 mmol) 1,4-Diazabicyclo[2.2.2]octan und 0,8 g (5,5 mmol) 3-Ethylaminomethyl-3-hydroxy-pyrrolidin versetzt und 1 Stunde unter Rückfluß erhitzt. Die Suspension wird eingeengt, der Rückstand abgesaugt, mit Wasser gewaschen und mit wenig halbkonzentrierter Salzsäure in Lösung gebracht. Durch Zugabe von Ethanol wird das Hydrochlorid ausgefällt. Man saugt ab, wäscht mit Ethanol und trocknet bei 100° im Vakuum.

Ausbeute: 1,3 g (56,5 % der Theorie) 8-Chlor-1-cyclopropyl-7-(3-ethylaminomethyl-3-hydroxy-1-pyrrolidinyl)-6-fluor-1,4-dihydro-4-oxo-3-chinolincarbonsäure-Hydrochlorid vom Schmelzpunkt 222-223° (unter Zersetzung)

Beispiel 3

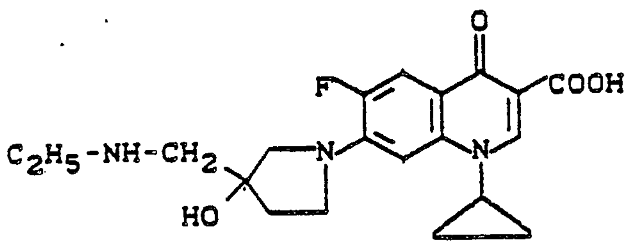

Analog Beispiel 1 wird 1-Cyclopropyl-6,7-difluor-1,4-dihydro-4-oxo-3-chinolincarbonsäure zu 1-Cyclopropyl-7-(3-ethylaminomethyl-3-hydroxy-1-pyrrolidinyl)-6-fluor-1,4-dihydro-4-oxo-3-chinolincarbonsäure vom Schmelzpunkt 202-207° umgesetzt.

Beispiel 4

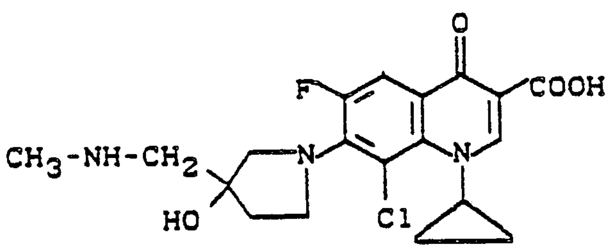

1,5 g (5 mmol) 8-Chlor-1-cyclopropyl-6,7-difluor-1,4-dihydro-4-oxo-3-chinolincarbonsäure werden in 10

ml Acetonitril und 5 ml Dimethylformamid mit 2,2 g (20 mmol) 1,4-Diazabicyclo[2.2.2]octan und 1,12 g (5,5 mmol) 3-Hydroxy-3-methylaminomethyl-pyrrolidin- Dihydrochlorid 1 Stunde unter Rückfluß erhitzt. Die Suspension wird eingeengt, mit Wasser verrührt und das ungelöste Produkt abgesaugt, mit Wasser gewaschen und getrocknet. Das erhaltene Rohprodukt (1,97 g) wird aus Dimethylformamid umkristallisiert.

Ausbeute: 1,55 g (75,7 % der Theorie) 8-Chlor-1-cyclopropyl-6-fluor-1,4-dihydro-7-(3-hydroxy-3-methylaminomethyl-1-pyrrolidinyl)-4-oxo-3-chinolincarbonsäure vom Schmelzpunkt 266-268° (unter Zersetzung).

Beispiel 5

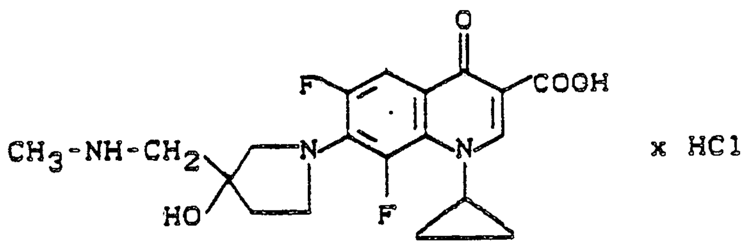

Analog Beispiel 4 setzt man 1-Cyclopropyl-6,7,8-trifluor-1,4-dihydro-4-oxo-3-chinolincarbonsäure um und löst das erhaltene Rohprodukt (1,75 g) in 10 ml halbkonzentrierter Salzsäure auf, fällt das Hydrochlorid durch Versetzen mit Ethanol aus, saugt ab, wäscht mit Ethanol und trocknet bei 100° im Vakuum.

Ausbeute: 1,2 g (56 % der Theorie) 1-Cyclopropyl-6,8-difluor-1,4-dihydro-7-(3-hydroxy-3-methylaminomethyl-1-pyrrolidinyl)-4-oxo-3-chinolincarbonsäure-Hydrochlorid vom Schmelzpunkt 274-276° (unter Zersetzung).

Beispiel 6

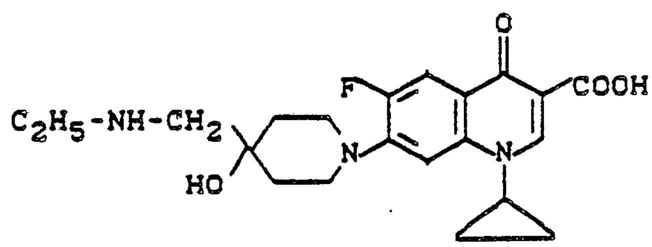

2,65 g (10 mmol) 1-Cyclopropyl-6,7-difluor-1,4-dihydro4-oxo-3-chinolincarbonsäure werden in einer Mischung aus 20 ml Acetonitril und 10 ml Dimethylformamid mit 3,3 g (30 mmol) 1,4-Diazabicyclo[2.2.2]-octan und 2,5 g (11 mmol) 4-Ethylaminomethyl-4-hydroxy-piperidindihydrochlorid versetzt und 1 Stunde unter Rückfluß erhitzt. Die Suspension wird eingeengt, der Rückstand mit Wasser verrührt (pH 7), der Niederschlag abgesaugt, mit Wasser gewaschen, getrocknet und aus Dimethylformamid umkristallisiert.

Ausbeute: 3,03 g (75,2 % der Theorie) 1-Cyclopropyl-7-(4-ethylaminomethyl-4-hydroxy-1-piperidinyl)-6-fluor1,4-dihydro-4-oxo-3-chinolincarbonsäure vom Schmelzpunkt 258-259° (unter Zersetzung).

Beispiel 7

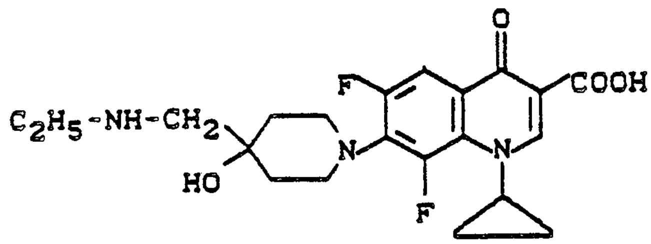

Man setzt analog Beispiel 6 1-Cyclopropyl-6,7,8-trifluor-1,4-dihydro-4-oxo-3-chinolincarbonsäure ein und erhält 1-Cyclopropyl-7-(4-ethylaminomethyl-4-hydroxy-1-piperidinyl)-6,8-difluor-1,4-dihydro-4-oxo-3-chinolincarbonsäure vom Schmelzpunkt 270-280° (unter Zersetzung).

Beispiel 8

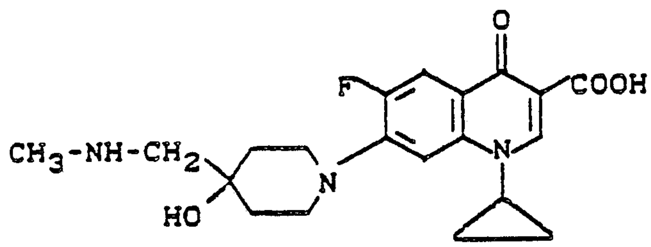

Analog Beispiel 6 wird 1-Cyclopropyl-6,7-difluor-1,4-dihydro-4-oxo-3-chinolincarbonsäure mit 4-Hydroxy-4-methylaminomethyl-piperidin-Dihydrochlorid zu 1-Cyclopropyl-6-fluor-1,4-dihydro-7-(4-hydroxy-4-methylaminomethyl-1-piperidinyl)-4-oxo-3-chinolincarbonsäure vom Schmelzpunkt 162° (unter Zersetzung) umgesetzt.

Beispiel 9

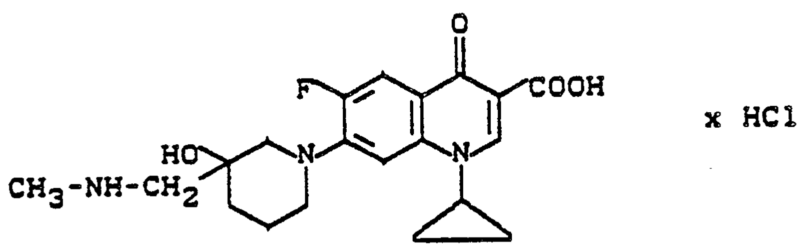

Analog Beispiel 2 wird 1-Cyclopropyl-6,7-difluor-1,4-dihydro-4-oxo-3-chinolincarbonsäure mit 3-Hydroxy-3-methylaminomethyl-piperidin zu 1-Cyclopropyl-6-fluor1,4-dihydro-7-(3-hydroxy-3-methylaminomethyl-1-piperidinyl)-4-oxo-3-chinolincarbonsäure-hydrochlorid vom Schmelzpunkt 293-296° (unter Zersetzung) umgesetzt.

Beispiel 10

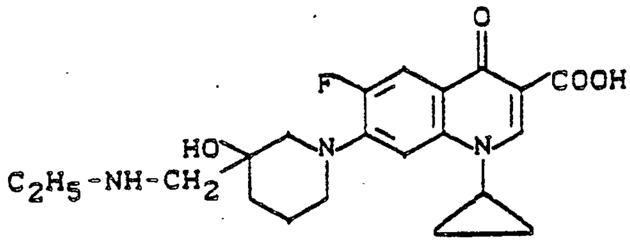

Analog Beispiel 1 wird 1-Cyclopropyl-6,7-difluor-1,4-dihydro-4-oxo-3-chinolincarbonsäure mit 3-Ethylaminomethyl-3-hydroxy-piperidin zu 1-Cyclopropyl-7-(3-ethylaminomethyl-3-hydroxy-1-piperidinyl)-6-fluor-1,4-dihydro-4-oxo-3-chinolincarbonsäure vom Schmelzpunkt 199-203° C (unter Zersetzung) umsetzt.

Beispiel 11

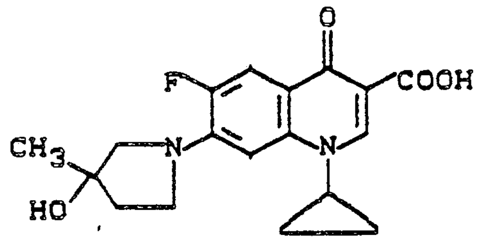

1,3 g (5 mmol) 1-Cyclopropyl-6,7-difluor-1,4-dihydro-4-oxo-3-chinolincarbonsäure werden in 10 ml Acetonitril und 5 ml Dimethylformamid mit 1,1 g (10 mmol) 1,4-Diazabicyclo[2.2.2]octan und 540 mg (5,4 mmol) 3-Hydroxy-3-methyl-pyrrolidin 1 Stunde unter Rückfluß erhitzt. Die Suspension wird eingeengt, der Rückstand mit Wasser verrührt, der Niederschlag abgesaugt, mit Wasser gewaschen, aus Dimethylformamid umkristallisiert und bei 100° im Vakuum getrocknet.

Ausbeute: 1,4 g (81 % der Theorie) 1-Cyclopropyl-6-fluor-1,4-dihydro-7-(3-hydroxy-3-methyl-1-pyrrolidinyl)4-oxo-3-chinolincarbonsäure vom Schmelzpunkt 315-320° (unter Zersetzung).

Beispiel 12

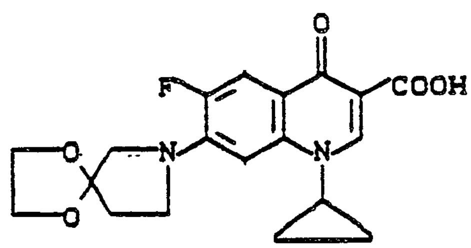

Analog Beispiel 11 wird 1-Cyclopropyl-6,7-difluor-1,4-dihydro-4-oxo-3-chinolincarbonsäure mit 1,4-Dioxa-7-azaspiro[4.4]nonan zu 1-Cyclopropyl-7-(1,4-dioxa-7-azaspiro[4.4]non-7-yl)-6-fluor-1,4-dihydro-4-oxo-3-chinolincarbonsäure vom Schmelzpunkt 229-230° umgesetzt.

Beispiel 13

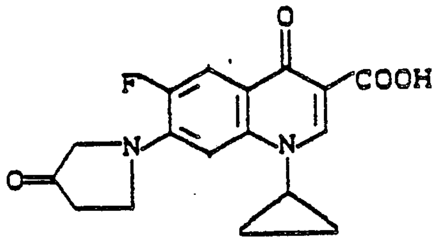

2 g 1-Cyclopropyl-7-(1,4-dioxa-7-aza-spiro[4.4]non-7-yl)-6-fluor-1,4-dihydro-4-oxo-3-chinolincarbonsäure werden in 100 ml Methanol suspendiert und mit 100 ml halbkonzentrierter Salzsäure 2 Stunden bei Raumtemperatur gerührt. Die Suspension wird eingeengt und der Rückstand aus Dimethylformamid umkristallisiert.

Ausbeute: 1 g (52 % der Theorie) 1-Cyclopropyl-6-fluor-1,4-dihydro-4-oxo-7-(3-oxo-1-pyrrolidinyl)-3-chinolincarbonsäure vom Schmelzpunkt 286-288° (unter Zersetzung).

Beispiel 14

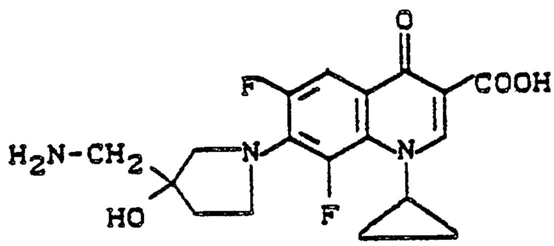

Man setzt entsprechend Beispiel 1 3-Aminomethyl-3-hydroxypyrrolidin zu 7-(3-Aminomethyl-3-hydroxy-1-pyrrolidinyl)-1-cyclopropyl-6,8-difluor-1,4-dihydro-4-oxo-3-chinolincarbonsäure vom Schmelzpunkt 248° C (unter Zersetzung) um.

Massenspektrum: $^{m}/e$ 379 ($M^+$), 361 (379-$H_2O$), 344 (361-F), 44 ($CO_2$), 41 ($C_3H_5$), 18 ($H_2O$).

Beispiel 15

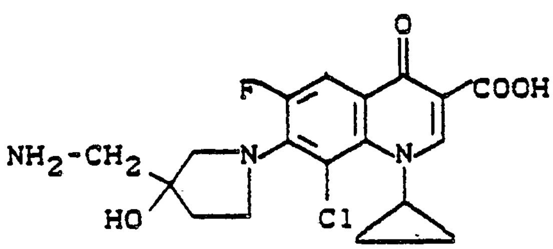

Man setzt analog Beispiel 14 mit 8-Chlor-1-cyclopropyl-6,7-difluor-1,4-dihydro-4-oxo-3-chinolincarbonsäure um, reinigt das erhaltene Produkt durch Chromatographie an Kieselgel mit Dichlormethan/Methanol/20 %-wäßriger Ammoniaklösung (2:4:1) als Laufmittel und erhält 7-(3-Aminomethyl-3-hydroxy-1-pyrrolidinyl)-8-chlor-1-cyclopropyl6-fluor-1,4-dihydro-4-oxo-3-chinolincarbonsäure vom Schmelzpunkt 240-243° C (unter Zersetzung).

FAB-Massenspektrum: m/e 396 [$(M+H)^+$], 368 [$(M+H-CO)^+$]

Beispiel 16

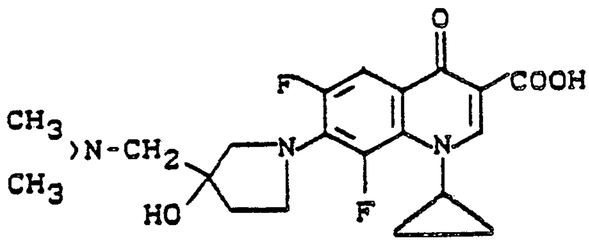

x HCl

1,42 g (5 mmol) 1-Cyclopropyl-6,7,8-trifluor-1,4-dihydroxy-4-oxo-3-chinolincarbonsäure werden in einer Mischung aus 10 ml Acetonitril und 5 ml Dimethylformamid und 0,8 g (5,6 mmol) 3-Hydroxy-3-dimethylaminomethyl-pyrrolidin und 1,1 g (10 mmol) 1,4-Diazabicyclo[2,2,2]octan 1 Stunde unter Rückfluß erhitzt. Die Suspension wird eingeengt, der Rückstand mit Wasser versetzt und mit verdünnter Salzsäure (1:1) angesäuert. Das auskristallisierende Salz wird abgesaugt und aus Dimethylformamid umkristallisiert.

Ausbeute: 1,1 g (50 % der Theorie) 1-Cyclopropyl-6,8-difluor-1,4-dihydro-7-(3-hydroxy-3-dimethylaminomethyl-1-pyrrolidinyl)-4-oxo-3-chinolincarbonsäure-Hydrochlorid vom Schmelzpunkt 292-295 °C (unter Zersetzung)

Beispiel 17

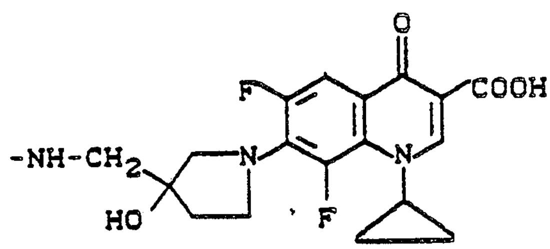

Analog Beispiel 6 wird 1-Cyclopropyl-6,7,8-trifluor-1,4-dihydro-4-oxo-3-chinolincarbonsäure mit 3-Cyclopropylaminomethyl-3-hydroxy-pyrrolidin-Dihydrochlorid zu 1-Cyclopropyl-7-(3-cyclopropylaminomethyl-3-hydroxy-1-pyrrolidinyl)-6,8-difluor-1,4-dihydro-4-oxo-3-chinolincarbonsäure vom Schmelzpunkt 161-162 ° (unter Zersetzung) umgesetzt.

Beispiel 18

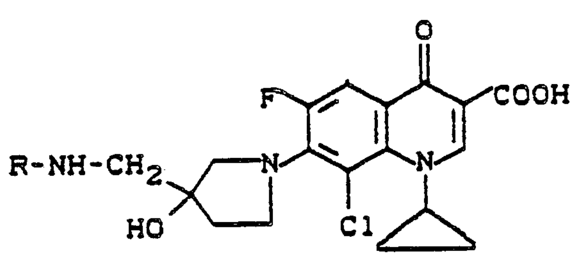

a) R = $(CH_3)_3C-O-CO$
b) R = H x HCl

a) Eine Mischung aus 6 g (20 mmol) 8-Chlor-1-cyclopropyl-6,7-difluor-1,4-dihydro-4-oxo-3-chinolincarbonsäure, 2,5 g (22,7 mmol) 1,4-Diazabicyclo[2.2.2]octan und 4,2 g (20 mmol) 3-tert.-Butoxycarbonylaminomethyl-3-hydroxy-pyrrolidin in 40 ml Acetonitril und 20 ml Dimethylformamid wird 3 Stunden unter Rückfluß erhitzt. Die Lösung wird im Vakuum eingeengt, der Rückstand mit Wasser verrührt, der ungelöste Niederschlag abgesaugt, mit Wasser gewaschen und getrocknet.

Ausbeute: 9,8 g (99 % der Theorie) rohes 7-(3-tert.-Butoxycarbonylaminomethyl-3-hydroxy-1-pyrrolidinyl)-8-chlor-1-cyclopropyl-6-fluor-1,4-dihydro-4-oxo-3-chinolincarbonsäure,
Schmelzpunkt: 192 °C (unter Zersetzung) (nach Umkristallisation aus Ethanol).

b) 9,5 g (19 mmol) des Produkts aus Beispiel 18a) werden in 300 ml halbkonzentrierter Salzsäure 30 Minuten bei Raumtemperatur gerührt. Die Mischung wird filtriert und das Filtrat bei 35 °C/12 mbar eingeengt. Der Rückstand wird aus Glykolmonomethylether umkristallisiert.

Ausbeute: 4,3 g (52 % der Theorie) 7-(3-Aminomethyl-3-hydroxy-1-pyrrolidinyl)-8-chlor-1-cyclopropyl-6-fluor-1,4-dihydro-4-oxo-3-chinolincarbonsäure-Hydrochlorid vom Schmelzpunkt 147-150 °C (unter Zersetzung); Gehalt: 93 %ig (nach HPLC).

Beispiel 19

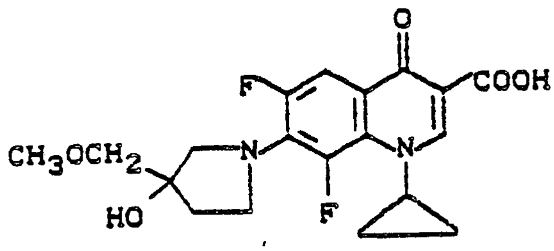

Man setzt analog Beispiel 1 mit 3-Hydroxy-3-methoxymethylpyrrolidin um und erhält 1-Cyclopropyl-6,8-difluor-1,4-dihydro-7-(3-hydroxy-3-methoxymethyl-1-pyrrolidinyl)-4-oxo-3-chinolincarbonsäure vom Schmelzpunkt 230-232° (unter Zersetzung) (aus Dimethylformamid umkristallisiert).

Beispiel 20

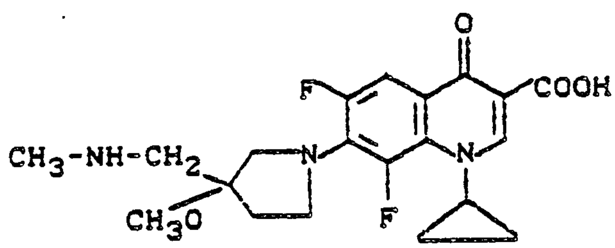

Man setzt analog Beispiel 1 mit 3-Methoxy-3-methylaminomethyl-pyrrolidin um und erhält 1-Cyclopropyl-6,8-difluor-1,4-dihydro-7-(3-methoxy-3-methylaminomethyl-1-pyrrolidinyl)-4-oxo-3-chinolincarbonsäure vom Schmelzpunkt 245-247 °C (unter Zersetzung) (aus Dimethylformamid umkristallisiert).

## Ansprüche

1. Chinolon- und Naphthyridoncarbonsäure-Derivate der Formel (I)

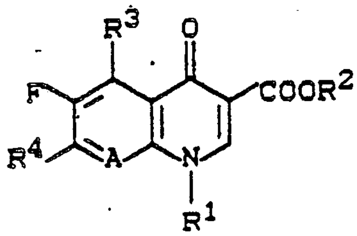

(I),

in welcher
$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl,
$R^3$ für Wasserstoff oder Amino,
$R^4$ für einen Rest der Formel

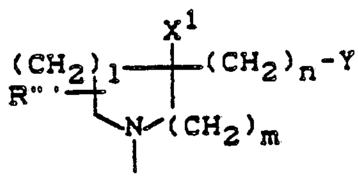

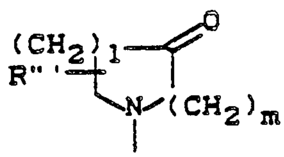

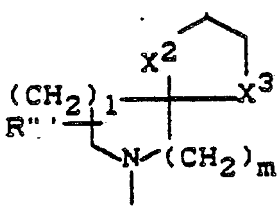

,

worin
l für 0, 1 oder 2,
m für 1 oder 2 steht, wobei l + m gemeinsam 1, 2 oder 3 sein können,
n für 1 oder 2,

Y für 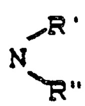 ,

OR, SR, Halogen oder Wasserstoff,

$X^1$ für 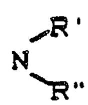 ,

OR, SR, Halogen, CN, $CONH_2$, COOH oder
$C_1$-$C_4$-Alkyl steht,
$X^2$ und $X^3$ gleich oder verschieden sein können und für Sauerstoff, Schwefel, NH oder N-$CH_3$ stehen,
R für Wasserstoff, $C_1$-$C_3$-Alkyl oder $C_1$-$C_3$-Acyl,
R′ für Wasserstoff, $C_1$-$C_3$-Alkyl, Allyl oder Propargyl und

$R''$ für Wasserstoff, $C_1$-$C_3$-Alkyl oder $C_3$-$C_6$-Cycloalkyl steht,
wobei
$R' + R''$ auch gemeinsam die Gruppen $-CH_2CH_2-O-CH_2CH_2-$ oder $-(CH_2)_k-$, in welcher k für 3, 4, oder 5 stehen kann, bedeutet,
$R'''$ für Wasserstoff oder $C_1$-$C_3$-Alkyl,
wobei 3-Amino-3-methyl-1-pyrrolidinyl für $R^4$ ausgenommen ist, und
A für N oder C-$R^5$ steht, worin
$R^5$ für Wasserstoff, Halogen wie Fluor oder Chlor, Methyl, Cyano oder Nitro steht oder auch gemeinsam mit $R^1$ eine Brücke der Struktur
$-O-CH_2-$ CH-CH$_3$, $-S-CH_2-$ CH-CH$_3$ oder $-CH_2-CH_2-$ CH-CH$_3$
bilden kann,
und deren pharmazeutisch verwendbaren Hydrate und Säureadditionssalze sowie die Alkali-, Erdalkali-, Silber- und Guanidiniumsalze der zugrundeliegenden Carbonsäuren.

2. Verbindungen der Formel (I) nach Anspruch 1, in der
$R^1$ für Ethyl, Isopropyl, Cyclopropyl, Vinyl, 2-Hydroxyethyl, 2-Fluorethyl, Amino, Methylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl,
$R^3$ für Wasserstoff oder Amino,
$R^4$ für einen Rest der Formel

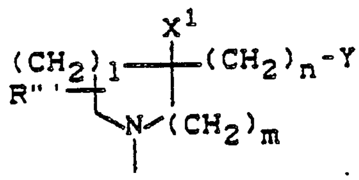
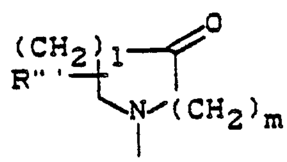
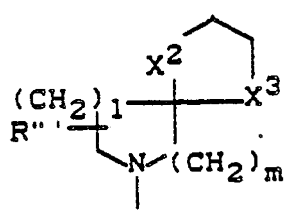
,

worin
l für 0, 1 oder 2,
m für 1 oder 2 steht, wobei l + m gemeinsam 1, 2 oder 3 sein können,
n für 1 oder 2,

Y für 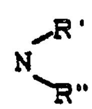 ,

OR, oder Wasserstoff,

$X^1$ für 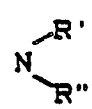 ,

OR, Fluor, Chlor oder $C_1$-$C_2$-Alkyl steht,
$X^2$ und $X^3$ gleich oder verschieden sein können und für Sauerstoff, Schwefel oder N-CH$_3$ stehen,
R für Wasserstoff, $C_1$-$C_2$-Alkyl oder Acetyl,
$R'$ für Wasserstoff oder $C_1$-$C_2$-Alkyl, und
$R''$ für Wasserstoff oder $C_1$-$C_2$-Alkyl steht,
wobei
$R' + R''$ auch gemeinsam die Gruppen $-CH_2CH_2-O-CH_2CH_2-$ oder $-(CH_2)_k$, in welcher k für 3, 4 oder 5

stehen kann, bedeutet,
R‴ für Wasserstoff oder $C_1$-$C_2$-Alkyl und
A für N oder C-$R^5$ steht, worin
$R^5$ für Wasserstoff, Halogen wie Fluor oder Chlor oder Methyl, steht oder auch gemeinsam mit $R^1$ eine Brücke der Struktur
-O-$CH_2$- CH-$CH_3$ oder -$CH_2$-$CH_2$- CH-$CH_3$
bilden kann,
und deren pharmazeutisch verwendbaren Hydrate und Säureadditionssalze sowie die Alkali-, Erdalkali-, Silber- und Guanidiniumsalze.

3. Verbindungen der Formel (I), nach Anspruch 1, in der
$R^1$ für Ethyl, Isopropyl, Cyclopropyl, Vinyl, 2-Hydroxyethyl, 2-Fluorethyl, Amino, Methylamino, Phenyl, 4-Fluorphenyl oder 2,4-Difluorphenyl,
$R^2$ für Wasserstoff oder Alkyl mit 1 oder 2 Kohlenstoffatomen,
$R^3$ für Wasserstoff,
$R^4$ für einen Rest der Formel

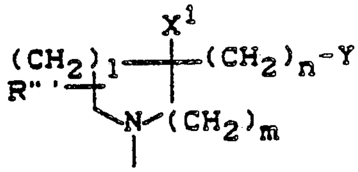

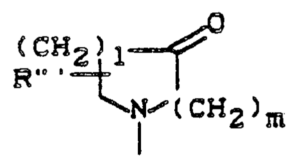

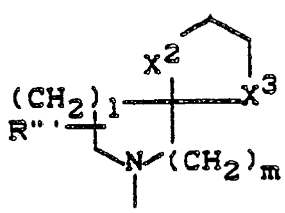
,

worin
l für 0, 1 oder 2,
m für 1 oder 2 steht, wobei l + m gemeinsam 1, 2 oder 3 sein können,
n für 1,

Y für 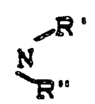 ,

OR, oder Wasserstoff,

$X^1$ für 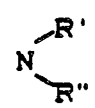 ,

OR, Chlor oder Methyl steht,
$X^2$ und $X^3$ gleich oder verschieden sein können und für Sauerstoff, oder N-$CH_3$ stehen,
R für Wasserstoff oder Methyl,
R′ für Wasserstoff oder Methyl,
R″ für Wasserstoff oder Methyl und
R‴ für Wasserstoff oder Methyl steht und
A für N oder C-$R^5$ steht, worin
$R^5$ für Wasserstoff, Halogen wie Fluor oder Chlor steht oder auch gemeinsam mit $R^1$ eine Brücke der Struktur
-O-$CH_2$- CH-$CH_3$,

bilden kann,
und deren pharmazeutisch verwendbaren Hydrate und Säureadditionssalze sowie die Alkali-, Erdalkali-, Silber- und Guanidiniumsalze.

4. Verfahren zur Herstellung der Verbindungen der Formel (I) nach Anspruch 1, dadurch gekennzeichnet, daß man Verbindungen der Formel (II)

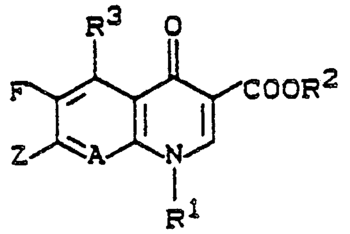
(II),

in welcher
Z für Fluor oder Chlor steht und
$R^1$, $R^2$, $R^3$ und A die im Anspruch 1 angegebene Bedeutung haben, mit Verbindungen der Formel (III)
$R^4$-H (III),
in welcher
$R^4$ die im Anspruch 1 angegebene Bedeutung hat, gegebenenfalls in Gegenwart von Säurefängern umsetzt.

5. Verfahren zur Herstellung der Verbindungen der Struktur (Ia)

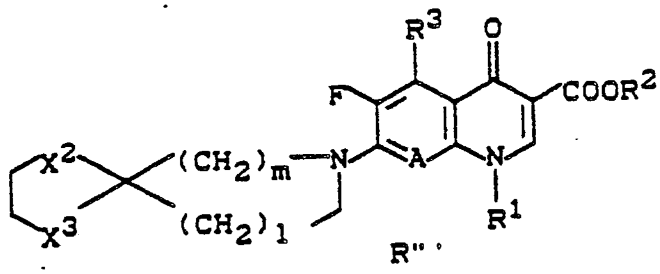
(Ia),

in welcher
$R^1$, $R^2$, $R^3$, $R'''$, A, $X^2$, $X^3$, m und l die im Anspruch 1 angegebene Bedeutung haben, dadurch gekennzeichnet, daß man Verbindungen der Formel (IV)

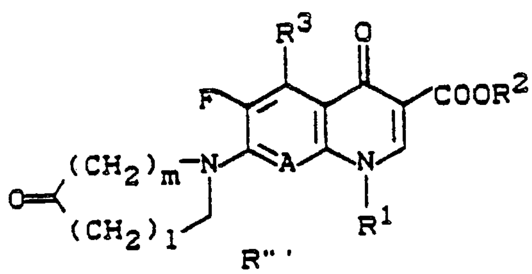
(IV),

in welcher
$R^1$, $R^2$, $R^3$, $R'''$, A, m und l die im Anspruch 1 angegebene Bedeutung haben, mit einer Verbindung der Formel (V)
$H$-$X^2$-$CH_2$-$CH_2$-$X^3$-$H$ (V),
in welcher
$X^2$ und $X^3$ die im Anspruch 1 angegebene Bedeutung haben, umsetzt.

6. Chinolon- und Naphthyridoncarbonsäure-Derivate der Formel (I) nach Anspruch 1 zur Anwendung in einem Verfahren zur therapeutischen Behandlung des menschlichen oder tierischen Körpers.

7. Arzneimittel, enthaltend Chinolon- und Naphthyridoncarbonsäure-Derivate der Formel (I) nach Anspruch 1.

8. Verwendung von Chinolon- und Naphthyridoncarbonsäure-Derivaten der Formel (I) nach Anspruch 1 zur Herstellung von Arzneimitteln.

9. Verbindungen der Formel

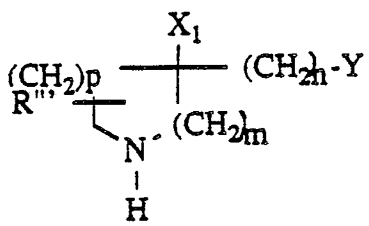 (III)

worin
l für 0, 1 oder 2,
m für 1 oder 2 steht, wobei l + m gemeinsam 1, 2 oder 3 sein können,
n für 1 oder 2,

Y für 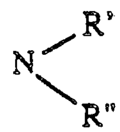 ,

OR, SR, Halogen oder Wasserstoff,

$X^1$ für 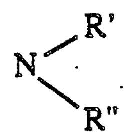 ,

OR, SR, Halogen, CN, $CONH_2$,
COOH oder $C_1$-$C_4$-Alkyl steht,
R für Wasserstoff, $C_1$-$C_3$-Alkyl oder $C_1$-$C_3$-Acyl,
R' für Wasserstoff, $C_1$-$C_3$-Alkyl, Allyl oder Propargyl und
R'' für Wasserstoff, $C_1$-$C_3$-Alkyl oder $C_3$-$C_6$-Cycloalkyl steht, wobei
R' + R'' auch gemeinsam die Gruppen
$-CH_2CH_2-O-CH_2CH_2-$ oder $-(CH_2)_k-$, in welcher k für 3, 4 oder 5 stehen kann, bedeutet,
R''' für Wasserstoff oder $C_1$-$C_3$-Alkyl,
wobei 3-Amino-3-methyl-1-pyrrolidinyl und 3-Hydroxy-3-aminomethyl-1-pyrrolidin ausgenommen sind.

10. Verbindungen der Formel III gemäß Anspruch 9 aus der Gruppe bestehend aus
3-Dimethylaminomethyl-3-fluorpyrrolidin,
3-Chlor-3-dimethylaminomethylpyrrolidin,
3-Fluormethyl-3-aminopyrrolidin,
3-Aminomethyl-3-fluorpyrrolidin,
3-Aminomethyl-3-chlorpyrrolidin,
3-Fluor-3-methylaminomethylpyrrolidin,
3-Chlor-3-methylaminomethylpyrrolidin,
3-Ethylaminomethyl-3-fluorpyrrolidin,
3-Chlor-3-ethylaminomethylpyrrolidin,
3-Hydroxy-3-methylpyrrolidin,
3-Hydroxy-3-methoxymethylpyrrolidin,
3-Methoxy-3-methylaminomethylpyrrolidin,
3-Ethoxy-3-ethylaminomethylpyrrolidin,
3-Chlor-3-ethylaminomethylpyrrolidin,
3-Ethylaminomethyl-3-fluorpyrrolidin,
3-Ethylaminomethyl-3-methylpyrrolidin,

3-Ethylaminomethyl-3-merkaptopyrrolidin,
3-Ethylaminomethyl-3-methylthiopyrrolidin,
3-Acetoxy-3-ethylaminomethylpyrrolidin,
3-Dimethylaminomethyl-3-hydroxypyrrolidin,
3-Hydroxy-3-pyrrolidinmethylpyrrolidin,
3-Hydroxy-3-morpholinmethylpyrrolidin,
3-Amino-3-ethylaminomethylpyrrolidin,
3-Acetylamino-3-ethylaminomethylpyrrolidin,
3-Ethylaminomethyl-3-methylaminopyrrolidin,
3-Dimethylamino-3-ethylaminomethylpyrrolidin,
3-Amino-3-hydroxymethylpyrrolidin,
3-Acetylamino-3-hydroxymethylpyrrolidin,
3-Amino-3-methoxymethylpyrrolidin,
3-tert.-Butoxycarbonylamino-3-methoxymethylpyrrolidin,
3-Amino-3-methylthiomethylpyrrolidin,
3-Amino-3-merkaptomethylpyrrolidin,
3-Cyclopropylaminomethyl-3-hydroxypyrrolidin,
3-Isopropylaminomethyl-3-hydroxypyrrolidin,
1,4-Dioxa-7-azaspiro[4.4]nonan,
1-Oxa-4,7-diazaspiro[4.4]nonan,
4-Methyl-1-oxa-4,7-diazaspiro[4.4]nonan,
1-Thia-4,7-diazaspiro[4.4]nonan,
1,4,7-Triazaspiro[4.4]nonan und
1,4-Dimethyl-1,4,7-triazaspiro[4.4]nonan.